(19) Europäisches Patentamt
European Patent Office
Office européen des brevets

(11) Veröffentlichungsnummer: **0 182 208**
**B1**

(12) # EUROPÄISCHE PATENTSCHRIFT

(45) Veröffentlichungstag der Patentschrift: 13.12.89

(21) Anmeldenummer: 85114160.6

(22) Anmeldetag: 07.11.85

(51) Int. Cl.⁴: C 07 H 15/24, A 61 K 31/70, C 12 P 19/56 // (C12P19/56, C12R1:54)

(54) **Urdamycine, Verfahren und Streptomyceten-Stamm zu ihrer Herstellung und ihre Verwendung.**

(30) Priorität: 16.11.84 DE 3441933

(43) Veröffentlichungstag der Anmeldung: 28.05.86 Patentblatt 86/22

(45) Bekanntmachung des Hinweises auf die Patenterteilung: 13.12.89 Patentblatt 89/50

(84) Benannte Vertragsstaaten: AT BE CH DE FR GB IT LI LU NL SE

(56) Entgegenhaltungen:

CHEMICAL & PHARMACEUTICAL BULLETIN, Band 29, Nr. 6, Juni 1981, Seiten 1788-1790, Pharmaceutical Society of Japan; NOBUTAKA IMAMURA et al.: "Identification of the aglycon part of vineomycin A1 with aquayamycin"

CHEMICAL & PHARMACEUTICAL BULLETIN, Band 32, Nr. 11, November 1984, Seiten 4350-4359, Pharmaceutical Society of Japan; KAZUHIKO OHTA et al.: "The absolute configuration of P-1894B, a potent prolyl hydroxylase inhibitor"

THE JOURNAL OF ANTIBIOTICS, Band 35, Nr. 5, Mai 1982, Seiten 602-608; NOBUTAKA IMAMURA et al.: "Biosynthesis of vineomycins A1 and B2"

THE JOURNAL OF ANTIBIOTICS, Band 38, Nr. 7, Juli 1985, Seiten 960-963, Japan Antibiotics Research Association, Tokyo, JP; Y. HAYAKAWA et al.: "Studies on the isotetracenone antibiotics. II. Kerriamycins A,B and C, new antitumor antibiotics"

THE JOURNAL OF ANTIBIOTICS, Band 38, Nr. 9, September 1985, Seiten 1171-1181, Japan Antibiotics Research Association, Tokyo, JP; T. UCHIDA et al.: "Saquayamycins, new aquayamycin-group antibiotics"

(73) Patentinhaber: HOECHST AKTIENGESELLSCHAFT
Postfach 80 03 20
D-6230 Frankfurt am Main 80 (DE)

(72) Erfinder: Zeeck, Axel, Prof. Dr.
Brüder-Grimm-Allee 22
D-3400 Göttingen (DE)
Erfinder: Rohr, Jürgen, Dr.
Zimmermannstrasse 64/106
D-3400 Göttingen (DE)
Erfinder: Zähner, Hans, Prof. Dr.
Im Hopfengarten 13
D-7400 Tübingen (DE)
Erfinder: Drautz, Hannelore, Dr.
Tulpenweg 16
D-7406 Mössingen (DE)
Erfinder: Sheldrick, George M. Prof. Dr.
Heinrich-Deppe-Ring 51
D-3406 Bovenden 1 (DE)
Erfinder: Jones, Peter, G. Dr.
Am Feldborn 4
D-3400 Göttingen (DE)
Erfinder: Paulus, Erich F. Dr.
Lindenweg 26
D-6239 Eppstein/Taunus (DE)

EP 0 182 208 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Vineomycine ($A_1$, $A_2$, $B_1$ und $B_2$) sind Benzanthrachinone und werden von Streptomyces matentsis subsp. vineus synthetisiert. Sie sind aktiv gegen gram-positive Bakterien und gegen Sarcoma 180 Tumor in Mäusen [Omura, S. et al. J. Antibiotics 30, 908 (1977) ; Imamura, N. et al., Chem. Pharm. Bull. 29, 1788 (1981) ; J. Antibiotics 34, 1517 (1981)]. Die Biosynthese dieser Stoffe wurde von Imamura, N. et al. untersucht und in J. Antibiotics 35, 602 (1982) beschrieben. Bisher bekannte Metabolite mit Benzanthrachinon-Struktur sind Aquayamycin [Sezaki, M. et al., Tetrahedron 26, 5171 (1970)], Rabelomycin [Liu, W. et al., J. Antibiotics 23, 437 (1970)], Ochromycinon [Bowie, J. et al., Tetrahedron letters 1967, 1499 (1967)], Tetrangulol und Tetrangomycin [Kunstmann, M.P. et al., J. Org. Chem. 31, 2920 (1970)], Yoronomycin (Ozaki, M. et al., Abstracts of the Annual Meeting of the Agricultural Chemical Society of Japan p. 225, 2 I-8, Kyoto 1976), und SS-228 Y [Kitahara, T. et al., J. Antibiotics 28, 280 (1975)].

Aus einer Bodenproche aus Tansania wurde ein Mikroorganismenstamm isoliert, der als Streptomyces fradiae identifiziert und bei der Deutschen Sammlung von Mikroorganismen (DSM), 3400 Göttingen, Grisebachstraße 8, unter der Hinterlegungs-Nr. DSM 3093 hinterlegt wurde. Dieser Stamm ist wie folgt charakterisiert :

| | |
|---|---|
| Sporenoberfläche : | Sm |
| Sporenmorphologie : | Sa |
| Chromogenität : | C- |
| Luftmycelfarbe : | cinnamomeus |

In einer eine Kohlenstoff- und Stickstoffquelle sowie die üblichen anorganischen Salze enthaltenden Nährlösung produziert der Stamm DSM 3093 als Urdamycine bezeichnete pharmakologisch wirksame Verbindungen. Diese Urdamycine sind also erhältlich durch Fermentation des Stammes DSM 3093 und Isolierung aus dem Mycel und insbesondere aus dem Fermentationsmedium.

Durch Extraktion des Kulturfiltrats mit einem organischen Lösemittel, das mit Wasser nicht oder nur wenig mischbar ist, erhält man farbige Extrakte, aus denen nach Abtrennen der lipophilen Bestandteile und Auftrennen des Rohproduktes durch Chromatographie sechs Fraktionen erhalten werden. Durch Chromatographie an einer Kieselgelsäule (Fließmittel : Chloroform/Methanol oder Methylenchlorid/Methanol) werdern beispielsweise die folgenden Fraktionen erhalten :

| Fraktion | Farbe | Urdamycin | Formel |
|---|---|---|---|
| 1 | gelb | B | II |
| 2 | rot | E | III |
| 3 | gelbrot | A | I |
| 4 | blau | D | - |
| 5 | rot | C | - |
| 6 | orange | F | IV |

Die chemischen Formeln, soweit sie ermittelt sind, sind in den Patentansprüchen wiedergegeben. Für Urdamycin C und D wurde bisher die Struktur noch nicht ermittelt ; diese Verbindungen sind durch eine Reihe physikalischer Daten charakterisiert (Beispiele).

Die Urdamycine wirken antibakteriell und tumorhemmend und können daher in Form pharmazeutischer Präparate zur Behandlung von bakteriell verursachten Infektionen oder zur Behandlung bei Tumorerkrankungen an Mensch und Tier verwendet werden.

Die Urdamycine sind Glykoside, aus denen sich in bekannter Weise, beispielsweise durch saure Hydrolyse oder Methanolyse, alle O-glykosidisch gebundenen Zucker abspalten lassen. Hierbei entsteht aus Urdamycin A das literaturbekannte Antibiotikum Aquayamycin (N. Imamura et al., Chem. Pharm. Bull. 29(6) 1788-1790 (1981)).

Die Urdamycine B bis F führen bei der sauren Hydrolyse bzw. Methanolyse zu neuen Aglykonen, die als Urdamycinone B bis F bezeichnet werden. Diese neuen Verbindungen sind ebenfalls antibakteriell und tumorhemmend.

Neben der Abspaltung der Zucker sind die Urdamycine und auch die daraus hergestellten Urdamycinone aufgrund ihrer funktionellen Gruppen in vielfältiger Weise chemischen Abwandlungen zugänglich, wobei Produkte erhalten werden können, die ebenfalls antibakterielle und tumorhemmende Wirkung zeigen.

Die Erfindung betrifft somit den Streptomyceten-Stamm DSM 3093, ein Verfahren zur Fermentation unter Verwendung dieses Stammes, wobei die Urdamycine A bis F gebildet werden, die Urdamycine A bis

F und deren Verwendung als antibakterielle bzw. tumorhemmende Mittel bzw. ihre Verwendung in antibakteriellen und tumorhemmenden Mitteln sowie als Zwischenprodukte zur Herstellung antibakterieller und tumorhemmender Substanzen. Weitere Aspekte der Erfindung und deren bevorzugte Ausgestaltungen sind in den Patentansprüchen niedergelegt bzw. im folgenden näher erläutert.

Anstelle des Stammes DSM 3093 können natürlich auch seine Mutanten und Varianten eingesetzt werden, soweit sie Urdamycin A bis F produzieren. Solche Mutanten können in an sich bekannter Weise durch physikalische Mittel, beispielsweise Bestrahlung wie mit Ultraviolett- oder Röntgenstrahlen, oder chemische Mutagene, wie beispielsweise Ethansulfonsäuremethylester (Ethylmethylsulfoncnat, EMS) oder 2-Hydroxy-4-methoxy-benzophenon (MOB) erzeugt werden.

Als bevorzugte Kohlenstoffquellen für die aerobe Fermentation eignen sich assimilierbare Kohlenhydrate und Zuckeralkohole wie Glucose, Lactose oder D-Mannit sowie kohlenhydrathaltige Naturprodukte, wie Malzextrakt. Als stickstoffhaltige Nährstoffe kommen in Betracht: Aminosäuren, Peptide und Proteine sowie deren Abbauprodukte wie Peptone oder Tryptone, ferner Fleischextrakte, gemahlene Samen, beispielswiese von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen anorganischen Salzen kann die Nährlösung beispielsweise Chloride, Carbonate, Sulfate oder Phosphate der Alkali- oder Erdalkalimetalle, Eisen, Zink und Mangan enthalten.

Die Bildung der Urdamycine A bis F verläuft besonders gut in einer Nährlösung, die etwa 2 % Fleischmehl, 10 % Malzextrakt und 1 % Calciumcarbonat, jeweils bezogen auf das Gewicht der gesamten Nährlösung, enthält.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 40 °C, vorzugsweise bei etwa 25 bis 30 °C, insbesondere bei 27 bis 28 °C erfolgen. Unter diesen Bedingungen zeigt die Kulturbrühe im allgemeinen nach 2 bis 4 Tagen eine nennenswerte antibiotische Wirkung.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1 :10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agrar, wachsen läßt.

Der Fermentationsverlauf kann anhand des pH-Wertes der Kultur, dem Mycelvolumen, durch Dünnschichtchromatographie oder Ausprüfen der biologischen Aktivität überwacht werden.

Die Isolierung der Urdamycine A bis F aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise auf Kieselgel mit Chloroform/Methanol als Laufmittel verwendet werden, wobei die Menge der gebildeten Antibiotika zweckmäßig mit einer Eichlösung verglichen wird.

Die Antibiotika können aus der unfiltrierten Kulturbrühe mit einem mit Wasser nicht oder nur wenig mischbaren organischen Lösemittel wie Chloroform oder Ethylacetat extrahiert werden. Da sich jedoch die Antibiotika nur zum geringen Teil im Mycel befinden, ist es vorteilhaft, die Kulturbrühe vom Mycel zu trennen, beispielsweise durch Zentrifugieren oder Filtrieren, vorzugsweise unter Zugabe von Filterhilfsmitteln, und die Antibiotika aus dem Überstand bzw. Filtrat zu isolieren, zweckmäßig im leicht sauren bis neutralen pH-Bereich, vorzugsweise bei pH 6 bis 7.

Zur Extraktion der Antibiotika aus dem Überstand bzw. Filtrat verwendet man zweckmäßig mit Wasser wenig oder nicht mischbare organische Lösemittel, insbesondere chlorierte Kohlenwasserstoffe, wie Chloroform oder Methylenchlorid oder Ester wie Ethylacetat. Die farbigen Extrakte werden, gegebenenfalls nach Eindampfen und Aufnehmen in einem polaren organischen Lösemittel, durch Fällen mit einem unpolaren Lösemittel, zweckmäßig einem Kohlenwasserstoff wie Petrolether, von stark lipophilen Bestandteilen befreit, die bis zu etwa 80 % des Gesamtextraktes ausmachen können. Aus dem Rückstand können die Urdamycine A bis F durch Chromatographie isoliert werden. Die Zusammensetzung der Urdamycin-Fraktionen kann je nach der Zusammensetzung des Nährmediums schwanken. Beispielsweise werden in einem Medium aus 2 % Malzextrakt, 2 % Destillationsrückstand aus der Ethanolgewinnung, 0,5 % Natriumchlorid und 0,1 % Natriumnitrat (jeweils Gew.-%) Urdamycin A und C in etwa gleicher Menge produziert, während in anderen Medien das Mengenverhältnis bis zu 6 :1 betragen kann.

Zur Isolierung der Urdamycine aus dem entfetteten Rohextrakt wird dieser zweckmäßig über eine Kieselgel-Säule gereinigt, wobei sich als Fließmittel Gemische aus niedermolekularen Chlorkohlenwasserstoffen und Alkanolen, beispielsweise Chloroform und Methanol im Volumenverhältnis 4 :1 oder Methylenchlorid und Methanol im Volumenverhältnis 85 :15, bewährt haben. Die in Form verschiedenfarbiger Zonen adsorbierten Komponenten werden nacheinander in Form verschiedenfarbiger Eluate eluiert, in denen jeweils eines der Urdamycine angereichert ist.

Zur Isolierung der reinen Urdamycine können die üblichen Verfahrensschritte Verwendung finden, wie Chromatographie, Gelfiltration oder Fällung aus ihren Lösungen in geeigneten organischen

Lösemitteln. Besonders bewährt hat sich eine Chromatographie an Kieselgel, wobei als Laufmittel eine Mischung aus einem niederen halogenierten Kohlenwasserstoff und einen niederen Alkanol, beispielsweise Methylenchlorid/Ethanol oder Chloroform/Methanol in einem Volumenverhältnis von beispielsweise 9 :1 Verwendung findet, gefolgt von einer Gelfiltration an geeigneten Medien wie Hydroxyalkoxypropyldextranen (lipophile SEPHADEX LH-Marken) mit einem niederen Alkanol wie Methanol und anschließendes Ausfällen, beispielsweise durch Eintropfen einer konzentrierten Lösung des Urdamycins in einem polaren organischen Lösemittel wie Aceton in ein unpolares Lösemittel, insbesondere einen Kohlenwasserstoff wie Petrolether oder n-Hexan.

Die reinen Urdamycine sind amorphe Feststoffe, die in Lösung in Abhängigkeit vom pH-Wert Indikatoreigenschaften haben. Die Verbindungen sind in stark polaren Lösemitteln wie Aceton, Methanol, Ethanol, Tetrahydrofuran oder Dimethylsulfoxid gut löslich, mäßig löslich in Chlorkohlenwasserstoffen wie Chloroform oder Methylenchlorid und unlöslichen Wasser oder Alkanen. Die Substanzen sind im festen Zustand und die Lösungen im pH-Bereich von 3 bis 9, insbesondere von 5 bis 8, stabil. Die Verbindungen lassen sich damit in übliche galenische Zubereitungen einarbeiten.

Die antibakterielle Wirkung kommt sowohl gegen grampositive wie gram-negative Bakterien zur Geltung, wie beispielsweise im Platten-Agardiffusiontest in vitro (10 µl/Testrondell, 6 mm Durchmesser) gezeigt werden kann, wo sich bei verschiedenen Keimen eine minimale Hemmkonzentration im Bereich von $\geqslant$ 0,03 bis $\leqslant$ 0,3 µg/ml sind beispielsweise: Achromobacter geminiani, Bacillus brevis, B. subtilis, Arthrobacter aurescens, A. crystallopoiete, Brevibacetrium flavum sowie verschiedene Streptomyceten, darunter z.B. S. antibioticus, S. violaceoreuber, S. prasinus, S. Lavendulae, glaucescens und S. viridochromogenes.

Die tumorhemmende Wirkung konnte an menschlichen und tierischen Tumorzellen in vitro gezeigt werden.

Die Überführung der Urdamycine in die entsprechenden Aglykone, die Urdamycinone, erfolgt in an sich bekannter Weise durch Hydrolyse oder Methanolyse in Gegenwart starker Säuren. Geeignet sind beispielsweise Gemische aus Methanol, Wasser und Trifluoressigsäure im Volumenverhältnis 2 :2 :1 oder Methanol, Wasser und konzentrierter Schwefelsäure im Volumenverhältnis 2 :1 :0, 1, in denen die reinen oder rohen Urdamycine 10 Minuten bis 3 Stunden bei Temperaturen von 0 bis 100 °C, vorzugsweise 20 bis 60 °C, insbesondere bei Zimmertemperatur, zur Reaktion gebracht werden. Die Reaktionsgemische werden dann mit Wasser verdünnt, beispielsweise mit dem etwa zehnfachen Volumen an Wasser, und mit einem mit Wasser wenig oder nicht mischbaren organischen Lösemittel, beispielsweise einem niederen Chlorkohlenwasserstoff oder Ester wie Chloroform oder Ethylacetat, extrahiert. Nach Auswaschen von restlicher Säure aus der organischen Phase mit Wasser und weitgehender oder vollständiger Abtrennung des organischen Lösemittels kann eine Reinigung durch Chromatographie oder vorteilhaft Gelfiltration erfolgen, wobei die gleichen Verfahrensschritte wie bei der Isolierung der Urdamycine Verwendung finden können.

Wie bereits erwähnt, ist das Urdamycinon A identisch mit dem Antibiotikum Aquayamycin. Die Erfindung eröffnet also einen neuen Weg zur Herstellung dieses Antibiotikums. Die Urdamycinone B bis F sind neue Verbindungen, die, wie erwähnt, ebenfalls antibakterielle und tumorhemmende Wirkung zeigen.

In den folgenden Beispielen wird die Erfindung näher erläutert. Prozentangaben beziehen sich auf das Gewicht, wenn nichts anderes angegeben ist. Mischungsverhältnisse bei Flüssigkeiten beziehen sich auf das Volumen, sofern keine anderen Angaben gemacht sind.

## Beispiel 1

a) Herstellung einer Sporensuspension des Produzentenstammes

100 ml Nährlösung (4 g Hefeextrakt, 10 g Malzextrakt, 4 g Glucose, 1 l Leitungswasser, pH-Wert vor dem Sterilisieren 7, 3, 20 g Agar zur Verfestigung) in einem 500 ml Erlenmeyerkolben mit einem seitlichen Einstich werden mit dem Stamm DSM 3093 beimpft und 72 Stunden bei 27 °C und 120 UpM auf der rotierenden Schüttelmaschine inkubiert. Anschliessend werden 20 ml Kulturflüssigkeit auf 50 ml Agar im 500 ml Erlenmeyerkolben gleichmäßig verteilt und dekantiert. Die Kulturen werden 10-14 Tage bei 27 °C inkubiert. Die nach dieser Zeit entstandenen Sporen eines Kolbens werden mit 500 ml entionisiertem Wasser, das einen Tropfen eines handelsüblichen nichtionischen Tensids enthält, abgeschwemmt und bei — 22 °C aufbewahrt.

b) Herstellung einer Kultur bzw. Vorkultur des Produzentenstammes im Erlenmeyerkolben

Ein 500 ml Erlenmeyerkolben mit einem seitlichen Einstich und 100 ml einer Nährlösung der Zusammensetzung 2 % Fleischmehl, 10 % Malzextrakt, 1 % Calciumcarbonat und Wasser ad 100 (pH 7,2 vor dem Autoklavieren) wird mit einer auf einem Schrägröhrchen gezogenen Kultur oder mit 0,2 ml Sporensuspension angeimpft und auf einer Schüttelmaschine bei 120 UpM und 27 °C inkubiert. Die maximale Antibiotikumproduktion ist nach 72 Stunden erreicht. Zum Animpfen von 10, 20, 25 und 100 l Fermentern genügt eine 48 Stunden alte Submerskultur (5 %) aus der gleichen Nährlösung.

Beispiel 2

Ein Fermenter von 10 l Fassungsvermögen wird unter folgenden Bedingungen betrieben : Bei 27 °C Inkubationstemperatur und 250 UpM des Rührers werden 4 Liter Luft pro Minute in die Kulturflüssigkeit (Medium wie in Beispiel 1a) eingeleitet. Durch wiederholte Zugabe weniger Tropfen ethanolischer Polyollösung kann die Schaumentwicklung unterdrückt werden. Das Produktionsmaximum wird nach ca. 48 Stunden erreicht. Die Ausbenten liegen bei ca. 10 mg/l Urdamycin A.

Zur Fermentation von Urdamycin C und D erweist sich folgende Nährlösung als optimal : 2 % Malzextrakt, 2 % Rückstand aus der Ethanoldestillation (Distiller solubile), 0,5 % NaCl, 0,1 % $NaNO_3$, pH-Wert vor dem Sterilisieren 7,3. Das Produktionsmaximum für die Urdamycine C und D liegt im Bereich von 72 bis 96 Stunden. Die Ausbeuten liegen bei 2-5 mg/l Kulturflüssigkeit.

Beispiel 3

Eine gemäß einem der vorhergehenden Beispiele erhaltene Kulturbrühe wird zur Isolierung der Urdamycine A bis F folgendermaßen aufgearbeitet :

Die Kulturbrühe wird durch eine Filterpresse unter Zusatz von 2 % Filterhilfsmittel (Diatomeenerde) filtriert. Das Mycel, das nur geringe antibiotische Aktivität aufweist, wird verworfen. Das Kulturfiltrat wird bei pH 7 erschöpfend mit Ethylacetat extrahiert. Die organische Phase wird getrocknet und eingedampft. Der ölige Verdampfungsrückstand wird mit reichlich Petrolether übergossen. Der ausgefallene Niederschlag wird abzentrifugiert und getrocknet und als Urdamycin-Rohprodukt weiter verarbeitet.

Das Rohprodukt wird an einer Kieselgel-Säule (Kieselgel 60, unter 0,08 mm) mit Chloroform/Methanol (4 :1) chromatographiert. Man eluiert nacheinander die folgenden Fraktionen, in denen jeweils eines der Urdamycine vornehmlich angereichert ist :

| | |
|---|---|
| Fraktion 1 (gelb) | Urdamycin B |
| Fraktion 2 (rot) | Urdamycin E |
| Fraktion 3 (gelbrot) | Urdamycin A |
| Fraktion 4 (blau) | Urdamycin D |
| Fraktion 5 (rot) | Urdamycin C |
| Fraktion 6 (orange) | Urdamycin F |

Beispiel 4

Die Fraktion 3 aus Beispeil 3 wurde an einer Kieselgelsäule mit Methylenchlorid/Ethanol (9 :1) und anschließend an einer Säule aus SEPHADEX LH 20 in Methanol nachgereinigt. Das jeweils aus der hauptzone eluierte Urdamycin A wird zuletzt in wenig Aceton gelöst und durch Eintropfen dieser Lösung in den 20-fachen Überschuß n-Hexan ausgefällt. Der so erhaltene gelbrote Feststoff zersetzt sich bei 160 °C und hat die folgenden Eigenschaften :

Zirkular-Dichroismus-Spektrum in Methanol :

$\lambda_{max}[\theta]^{24}$= 456 (+ 5000), 400 (— 9000), 328 (+ 35000), 290 sh (+ 11000), 261 (— 5000), 232 nm (+ 38000).

Die Elementaranalyse des bei 60 °C im Hochvakuum getrockneten Urdamycin A ergibt
C = 60,47 %, H = 6,66 %, kein Stickstoff und Schwefel nachweisbar.
Mol.-Masse : 845 gemäß FAB-Massenspektrum (negative Ionen)
Das Elektronenspektrum zeigt folgende Maxima :

Ethanol (gelbrot) : $\lambda_{max}(\varepsilon)$ = 440 sh (5400), 426 (5500), 319 nm (4500).
Ethanol/NaOH (ultramarinblau) : $\lambda_{max}$ 580 (5400), 404 (1700), 325 nm (8100)
IR (KBr) : 3430 ; 1728 ; 1657 sh ; 1650 ; 1639 ; 1620 ; $1580^{-1}$
UV (Ethanol) : $\lambda_{max}(\varepsilon)$ = 440 sh (5400) ; 426 (5500) ; 319 (4500) nm
(Ethanol/HCl) : $\lambda_{max}(\varepsilon)$ = wie Ethanol
(Ethanol/NaOH) : $\lambda_{max}(\varepsilon)$ = 580 (5400) ; 404 (1700) ; 325 (8100) nm

$^1$H-NMR (200 MHz, $d_6$-Aceton) : δ = 0,51 (d, J = 6,5 Hz, 5C-$CH_3$) ; 1,16 (d, J = 6,5 Hz, 5A-$CH_3$) ; 1,17 (s, 3-$CH_3$) ; 1,24 (d, J = 6 Hz, 5B-$CH_3$) ; 1,33 (o, J = 3 x ca. 12 Hz, 3'-Ha) ; 1,38 (d, J = 6 Hz, 6'-$CH_3$) ; 1,4-2,0 (komplex, 8H, 2C-$H_2$, 3C-$H_2$, 2A-$H_2$, 3A-$H_2$) ; 1,95 u. 2,04 (dd u. d, J = 15 u. 2 bzw. 15 Hz, 4-$H_2$) ; 2.20 (o, J = 13,5, 2 Hz, 3'-$H_e$) ; 2,37 (0, J = 13, 12, 12 Hz, 2B-$H_a$) ; 2,55 (o, J = 13, 5, 2 Hz, 2B-$H_e$) ; 2,56 u. 2,90 (dd u. d, J = 13, 2 bzw. 13 Hz, 2-$H_2$) ; 2,94 (o, J = 9, 9, 4 Hz, 5'-H**) ; 3,19 (o, J = 9, 9, 4 Hz, 4B-H**) ; 3,27 (dq, J = 9u. 6 Hz, 5B-H) ; 3,28 (o, J = 3 × 2 Hz, 4C-H) ; 3,53 (dq, J = 9 u. 6 Hz, 6'-H) ; 3,56 (m, 3B-H) ; 3,58 (o,

** Signale werden nach $CD_3OD$-Austausch zu dd, J = 9 u. 9 Hz

J = 3 × ca. 2 Hz, 4A-H) ; 3,68 (dq, J = 6,5 u. 2 Hz, 5C-H) ; 3,81 (s, OH*) ; 3,82 (0, J = 12, 9, 5 Hz, 4'-H) ; 4,03 (d, J = 5 Hz, OH*) ; 4,09 (d, J = 5 Hz, OH*) ; 4,24 (dq, J = 6,5 u. 2 Hz, 5A-H) ; 4,42 (s, OH*) ; 4,63 (d, J = 4 Hz, OH*) ; 4,64 (dd, J = 10 u. 2 Hz, 1B-H) ; 4,93 (dd, J = 10 u. 2 Hz, 2'H) ; 5,02 (d, J = 2 Hz, 1A-H) ; 5,32 (d, J = 3 Hz, OH*) ; 5,33 (d, J= 2 Hz, 1C-H) ; 6,53 (d, J = 10 Hz, 5-H) ; 6,93 (d, J = 10 Hz, 6-H) ; 7,68 (d, J = 8 Hz, 10-H) ; 7,99 (d, J = 8 Hz, 11-H) ; 12,38 (s, 8-OH*) ppm

* Signale verschwinden nach Austausch mit $CD_3OD$

(200 MHz, $d_6$-DMSO) : δ = 0,38 (d, J = 6,5 Hz, 5C-$CH_3$) ; 1,04 (d, J = 6,5 Hz, 5A-$CH_3$) ; 1,08 (s, 3-$CH_3$) ; 1,14 (d, J = 6 Hz, 5B-$CH_3$) ; ca. 1,2 (verd., 3'-$H_a$) ; 1,3-1,8 (kompl., 8H, 2C-, 3C-, 2A- u. 3A-$H_2$) ; 1,75 u. 1,86 (2d, J = je 15 Hz, 4-$H_2$) ; 2,00 (o, J = 13, 5, 2 Hz, 3'-$H_e$) ; 2,14 (o, J = 13, 10, 10 Hz, 2B-$H_a$) ; 2,39 u. 2,74 (2d, J = je 13 Hz, 2-$H_2$) ; ca. 2,5 (verd., 2B-$H_e$) ; 2,72 (dd, J = 2 × 10 Hz, 5'-H) ; 3,04 (dd, J = 2 × 10 Hz, 4 B-H) ; 3,10 (dq, J = 10 u. 6 Hz, 5B-H) ; 3,22 (o, J = 12, 9, 5 Hz, 4'-H) ; 4,13 (dq, J = 6,5 u. 2 Hz, 5A-H) ; 4,30 (d, J = 6 Hz, OH*) ; 4,47 (dd, J = 10 u. 2 Hz, 1B-H) ; 4,82 (dd, J = 10 u. 2 Hz, 2'-H) ; 4,84 (d, J = 5 Hz, OH*) ; 4,85 (s, 1A-H) ; 4,90 (d, J = 6 Hz, OH*) ; 5,10 (s, OH*) ; 5,11 (d, J = 5 Hz, OH*) ; 5,12 (s, 1C-H) ; 5,65 (s, OH*) ; 6,39 (d, J = 10 Hz, 5-H) ; 6,73 (d, J = 10 Hz, 6-H) ; 7,55 (d, J = 8 Hz, 10-H) ; 7,84 (d, J = 8 Hz, 11-H) ; 12,26 (s, 8-OH*) ppm.

* Signale verschwinden nach $D_2O$-Austausch.

CD (Methanol) : $\lambda_{extr.}$ ($\theta^{24} \times 10^{-4}$) = 456 (+ 0,5) ; 400 (— 0,9) ; 328 (+ 3,5) ; 290sh (+ 1,1) ; 261 (— 0,5) ; 232 (+ 3,8) nm
$\alpha_D^{24}$ (c = 0,1 Aceton) = + 32°

Beispiel 5 : (Urdamycin B)

450 mg der gelben Fraktion aus der Vortrennung gemäß Beispiel 3 werden an Kieselgel (Chloroform/Methanol = 9 :1) und SEPHADEX LH 20 (Methanol) chromatographiert. Man erhält nach Eintragen der gesättigten Aceton-Lösung in n-Hexan neben 15 mg Urdamycin E 370 mg Urdamycin B, als gelben, amorphen Feststoff.

IR (KBr) : 3400 ; 1750sh ; 1705sh ; 1694 ; 1668 ; 1626 ; 1589 $cm^{-1}$
UV (Methanol) : $\lambda_{max}(\varepsilon)$ = 403 (3900) ; 268 (27200) nm
(Methanol/HCl) : $\lambda_{max}(\varepsilon)$ = wie Methanol
(Methanol/NaOH) : $\lambda_{max}(\varepsilon)$ = 505 (3600) ; 315 sh (3800) ; 266 (17300) ; 248 (14100) nm
$^1$H-NMR (200 MHz, $d_6$-DMSO) :

| | | Urdamycin B[a)] | Urdamycinon B[b)] |
|---|---|---|---|
| 2-$H_a$ | : | 3,24 d (17) | 3,33 d (17) |
| 2-$H_e$ | : | 3,08 dd (17, 2) | 3,21 dd (17, 1) |
| 3-$CH_3$ | : | 1,35 s | 1,49 s |
| 4-$H_a$ | : | 3,07 d (15) | 3,10 d (14,5) |
| 4-$H_e$ | : | 2,74 dd (15, 2) | 2,90 dd (14,5, 1,5) |
| 5-H | : | 7,57 d (8) | 7,61 d (8) |
| 6-H | : | 7,89 d (8) | 7,95 d (8) |
| 10-H | : | 7,76 d (8) | 7,76 d (8) |
| 11-H | : | 8,26 d (8) | 8,31 d (8) |
| 2'-H | : | 4,83 dd (10, 2) | 4,92 dd (11,5, 1,5) |
| 3'-$H_a$ | : | ca. 1,2, verdeckt | 1,23 o (3x ca. 12)[c)] |
| 3'-$H_e$ | : | 2,46 o (13, 5, 2) | 2,46 o (13, 5, 2) |
| 4'-H | : | 3,70 o (12, 9, 5) | 3,76 o (12, 9,5, 5) |
| 5'-H | : | 3,07 dd [a] | 3,11 dd (8,5, 8) |
| 6'-H | : | ca. 3,4, komplex | 3,50 dq (9,5, 6) |

(Fortsetzung)

| | Urdamycin B[a)] | Urdamycinon B[b)] |
|---|---|---|
| 6'-$CH_3$ : | 1,29 d (6) | 1,37 (6) |
| OH-Signale:[b)] | 4,85 d (5) | 3,82 s |
| | 4,92 d (5) | 4,22 breit |
| | 5,04 s (3-OH) | 4,22 breit |
| | 5,13 d (5) | |

CD (Methanol) : $\lambda_{extr.}(\theta^{20} \times 10^{-4})$ = 415 (+ 0,2) ; 375 (+ 0,03) ; 330 (+ 0,2) ; 271 (— 1,8) ; 227 (+ 0,8) nm

Beispiel 6 : (Urdamycin C)

400 mg der zweiten, größeren roten Fraktion aus der Vortrennung nach Beispiel 3 werden durch Chromatographie an Kieselgel (Methylenchlorid/Ethanol = 9 :1) und SEPHADEX LH 20 (Methanol) gereinigt und als Feststoff durch Eintropfen einer gesättigten Aceton-Lösung in n-Pentan ausgefällt. Ausbeute 310 mg, $R_f$ : $R_f$-Wert auf Kieselgel-Dünnschichtplatten (20 × 20 cm, 0,25 mm Schichtdicke, Laufstrecke 15 cm) von 0,51 mit Chloroform/Methanol (4 :1 Volumen) bzw. 0,15 mit Methylenchlorid/Ethanol (9 :1 Volumen).

| | | | |
|---|---|---|---|
| $C_{51}H_{60}O_{19}$ (9.77.04) | Ber. | C 62,7 | H 6,2 |
| Fp : 200 °C (Zersetzung) | Gef. | C 63,4 | H 6,7 |

IR (KBr) : 3420, 1732, 1705sh ; 1640 ; 1605 $cm^{-1}$
UV (Methanol) : $\lambda_{max}(\varepsilon)$ = 510 (12600) ; 410sh (6300) ; 308 (14800) ; 237sh (23600) ; 226 (25300) nm
(Methanol/HCl) : $\lambda_{max}(\varepsilon)$ = 510 (12600) ; 310 (15400) : 226 (26900) nm
(Methanol/NaOh) : $\lambda_{max}(\varepsilon)$ = 625sh (10400) ; 600 (12100) ; 414 (5500) ; 326 (12000) ; 238 (30000) nm

$^1$H-NMR (200 MHz, $d_6$-Aceton) : δ = 0,39 (d, J = 6,5 Hz, 5C-$CH_3$) ; 1,12 (s, 3-$CH_3$) ; 1,13 (d, J = 6,5 Hz, 5A-$CH_3$) ; 1,22 (d, J = 6 Hz, 5B-$CH_3$) ; 1,23 (d, J = 6 Hz, 6'-$CH_3$) ; 1,2-1,6 (kompl., 2C-$H_2$, 3C-$H_2$, 2A-$H_2$, 3A-$H_2$) ; 1,8-2,2 (kompl., verd., u.a. 4-$H_2$) ; 2,18 (o, J = 13,5,2 Hz, 3'-$H_e$) ; 2,58 (o, J = 13,5,2 Hz, 2B—$H_e$) ; 2,70 u. 3,16 (dd u. d, J = 13,2 u. 13 Hz, 2-$H_e$ u. 2-$H_a$) ; 2,92 (verd.***, 5'-H) ; 3,06 (o, J = 9,9,4,5 Hz, 4B-H**) ; 3,33 (s, breit, 4C-H) ; ca. 3,3 (verd. 5B-H ; 3,48 (dq, J = 9 u. 6 Hz, 6'-H) ; ca. 3,5 (verd., 3B-H) ; 3,55 (s, breit, 4A-H) ; 3,63 (dq, J = 6,5 u. 2 Hz, 5C-H) ; 3,79 (o, J = 12, 9 u. 5 Hz, 4'-H) ; 3,80 (s, OH*) ; 4,05 (d, J = 4,5 Hz, OH) ; 4,10 (d, J = 4,5 Hz, OH*) ; 4,20 (dq, J = 6,5 u. 2 Hz, 5A-H) ; 4,56 (s, OH*) ; 4,59 (d, J = 3,5 Hz, OH*) ; 4,60 (dd, J = 10 u. 1,5 Hz, 1B-H) ; 4,75 (dd, J = 10 u. 1,5 Hz ; 2'-H) ; 5,00 (s, 1-H) ; 5,29 (s, OH*) ; 5,44 (s, 1C-H) ; 6,20 (d, 1H, J = 10 Hz) ; 7,05 (d, 1H, J = 10 Hz) ; 7,06 (2d, 2H, J = je 8 Hz) ; 7,47 (2d, 2H, J = 8 Hz) ; 7,96 (d, 1H, J = 2 Hz) ; 9,10 (s, OH*) ; 13,16 (s, OH*) ppm(200 MHz, $d_3$-Acetonitril) ; δ = 0,51 (d, J = 6,5 Hz, 5C-$CH_3$) ; 1,22 (s, 3-$CH_3$) ; 1,24 (d, J = 6,5 Hz, 5A-$CH_3$) ; 1,32 (2d, J = je 6 Hz, 5B u. 6'-$CH_3$) ; 1,4-1,8 (komplex, 8H) ; 1,8-2,1 (kompl., verd., u.a. 4-$H_2$) ; ca. 2,3 (verd., 3'-$H_e$) ; 2,56 (d, J = 6 Hz, OH*) ; 2,60 (o, J = 13,5,2 Hz, 2B-$H_e$) ; 2,72 u. 3,14 (dd u. d, J = 13 u. 2 bzw. 13 Hz, 2-$H_2$) ; 2,97 (o, J = 9,9,5 Hz, 5'-H**) ; 3,15 (o, J = 9,9,4 Hz, 4B-H**) ; 3,30 (dq, J = 9u. 6 Hz, 5B-H) ; 3,46 (s, breit, 4C-H) ; ca. 3,5 (verd., 3B-H) ; 3,56 (dq, J = 9 u. 6 Hz, 6'-H) ; 3,63 (s, breit, 4A-H) ; 3,71 (dq, J = 6,5 u. 1 Hz, 5C-H) ; 3,82 (o, J = 12,9,5 Hz, 4'-H) ; 4,07 (d, J = 3,5 Hz, OH*) ; 4,21 (dq, J = 6,5 u. 2 Hz, 5A-H) ; 4,41 (s, OH*) ; 4,51 (d, J = 2 Hz, OH*) ; 4,66 (dd, J = 10 u. 2 Hz, 1B-H) ; 4,83 (dd, J = 10 u. 1,5 Hz, 2'-H) ; 5,08 (s, 1A-H) ; 5,46 (s, 1C-H) ; 6,24 (d, 1H, J = 10 Hz) ; 7,13 (3d, 3H, J = 10,8,8 Hz) ; 7,51 (2d, J = je 8 Hz, 2H) ; 7,69 (s, OH*) ; 7,89 (d, 1H, J = 1,5 Hz ; 13,14 (s, OH*) ppm

* Signale verschwinden nach $D_2O$-Austausch,
** Signale werden nach $D_2O$-Austausch zum dd, die kleine Kopplung ($J_{H-OH}$) entfällt,
*** Signal ist nach $D_2O$-Austausch als dd, J = 9 u. 9 Hz, sichtbar.

$^{13}$C-NMR (50,3 MHz, $CD_3CN$, DEPT) : δ = 16,4 (0, C-6C) ; 16,8 (o, C-6A) ; 17,9 (o, C-6B) ; 18,2 (o, C-7') ; 23,8 (u, C-3C) ; 24,7 (u. C-3A) ; 25,0 (u, C-2A) ; 25,7 (u, C-2C) ; 29,4 (o, C-13) ; 36,9 (u, C-C-2B) ; 39,6 (u, C-3') ; 43,3 (u, C-4) ; 54,2 (u, C-2) ; 66,5 (o, C-4C) ; 67,0 (o, C-4A) ; 67,1 (o, C-5C) ; 71,4 (o, C-5B) ; 71,5 (o, C-2') ; 72,0 (o, C-6') ; 74,6 (—, C-3) ; 75,9 (o, C-5A) ; 76,1 (o, C-3B) ; 76,4 (o, C-4B) ; 77,7 (o, C-4') ; 78,4 (o, c-5') ; 82,0 (—, C-4a) ; 82,7 (—, C-12b) ; 95,2 (o, C-1A) ; 95,5 (o, C-1C) ; 101,7 (o. C-1B) ; 112,3 (—, C-7a) ;

115,6 (o) ; 115,9 (—) ; 118,3 (o) ; 123,6 (—) ; 125,0 (—) ; 127,9 (—) ; 132,3 (—) ; 133,9 (—) ; 133,8 (o) ; 134,1 (o) ; 138,1 (o) ; 143,1 (—, C-9 ?) ; 144,7 (—) ; 156,2 (—, c-8 ?) ; 159,1 (—) ; 159,6 (—) ; 187,2 (—, C-7 ?) ; 204,5 (—, C-1) ppm

CD (Methanol) : $\lambda_{extr.}(\theta^{21} \times 10^{-4})$ = 500 (—0,5) ; 336 (+1,7) ; 283 (—1,2) ; 248 (+2,9) ; 236 (+2,2) ; 223 (+4,8) nm

Beispiel 7 : (Urdamycin D)

240 mg der blauen Fraktion aus der Vortrennung nach Beispiel 3 werden an Kieselgel (Methylenchlorid/Methanol = 85 :15) und SEPHADEX LH 20 (Methanol) chromatographiert. Man erhält 85 mg Urdamycin D, 18 mg Urdamycin A und 50 mg Urdamycin C. Urdamycin D wird in wenig Aceton aufgenommen und als blaues, amorphes Pulver aus n-Hexan ausgefällt, $R_f$ : $R_f$-Wert auf Kieselgel-Dünnschichtplatten (20 × 20 cm, 0,25 mm Schichtdicke, Laufstrecke 15 cm) von 0,52 mit Chloroform/Methanol (4 :1 Volumen) bzw. 0,17 mit Methylenchlorid/Ethanol (9 :1 Volumen).

| | | | | | |
|---|---|---|---|---|---|
| $C_{54}H_{65}NO_{17}$ (1000,12) ? | Ber. | C 64,9 | H 6,6 | N 1,4 |
| Fp. : 220°C (Zersetzung) | Gef. | C 64,1 | H 6,5 | N 1,5 |

IR (KBr) : 3420 ; 1735 ; 1710 sh ; 1690 ; 1668 ; 1595 $cm^{-1}$
UV (Methanol) : $\lambda_{max}(\varepsilon)$ = 575 (11200) ; 328 (11600) nm
(Methanol/HCl) : $\lambda_{max}(\varepsilon)$ = wie Methanol
(Methanol/NaOH) : $\lambda_{max}(\varepsilon)$ = 635 sh (7200) ; 600 (8500) ; 412 (4200) ; 324 (7500) ; 270sh (17600) ; 222 (32400) nm

$^1$H-NMR (200 MHz, $d_6$-Aceton) : δ = 0,41 (d, J = 6,5 Hz, 5C-$CH_3$) ; 1,07 (d, J = 6 Hz, 5B-$CH_3$) ; 1,12 (s, $CH_3$) ; 1,13 (d, J = 6,5 Hz, 5A-$CH_3$) ; 1,20 (s, 3-$CH_3$) ; 1,22 (d, J = 6 Hz, 6'-$CH_3$) ; 1,2-1,6 (komplex. 2C-$H_2$, 2A-$H_2$, 3C-$H_2$, 3A-$H_2$) ; 1,8-2,2 (komplex, u.a. 4-$H_2$, 3'-$H_e$) ; 2,58 (o, J = 13,5,2 Hz, 2B-$H_e$) ; 2,70 u. 3,20 (dd u. d, J = 13,2 u. 13 Hz, 2-$H_2$) ; 2,91 (dd, J = 9 u. 9 Hz, 5'-H) ; 2,95 (dd, J = 9 u. 9 Hz, 4B-H) ; 3,24 (dq, J = 9 u. 6 Hz, 5B-H) ; 3,26 (br. s, 4C-H) ; 3,42 (dq, J = 9 u. 6 Hz, 6'-H) ; 3,53 (m, 3B-H) ; 3,57 (br. s, 4A-H) ; 3,64 (dq, J = 6,5 u. 1 Hz, 5C-H) ; 3,77 (o, J = 12,9,5 Hz, 4'-H) ; 4,17 (dq, J = 6,5 u. 1,5 Hz, 5A-H) ; 4,59 (dd, J = 10 u. 2 Hz, 1B-H) ; 4,60 (breit, OH*) ; 4,77 (dd, J = 10 u. 1,5 Hz, 2'-H) ; 5,00 (d, J = 1,5 Hz, 1A-H) ; 5,26 (breit, OH*) ; 5,46 (d, J = 1 Hz, 1C-H) ; 6,16 (d, 1H, J = 10 Hz) ; 7,06 (d, 1H, J = 10 Hz) ; 7,20 (dt, 1H, J = 7,5 u. 1,5 Hz) ; 7,29 (dt, 1H, J = 7,5 u. 1,5 Hz) ; 7,62 (dd, 1H, J = 7,5 u. 1,5 Hz) ; 7,63 (dd, 1H, J = 7,5 u. 1,5 Hz) ; 8,12 (s, 1H) ; 8,25 (d, J = 1,5 Hz, 1H) ; 11,26 (breit, OH*) ppm
(200 MHz), ($d_8$-Dioxan) : δ = 0,37 (d, J = 6,5 Hz, 5C-$CH_3$) ; 1,05 (d, J = 6 Hz, 5B-$CH_3$) ; 1,08 (s, $CH_3$) ; 1,10 (d, J = 6,5 Hz, 5A-$CH_3$) ; 1,12 (s, 3-$CH_3$) ; 1,21 (d, J = 6 Hz, 6'-$CH_3$) ; 1,26-1,8 (komplex, 2C-, 3C-, 3A-$H_2$) ; 1,8-2,2 (komplex, u.a. 4-$H_2$) ; 2,12 (verd. 3'-$H_e$**) ; 2,50 (o, J = 13,5,2 Hz, 2B-$H_e$**) ; 2,65 u. 3,10 (dd, J = 13 u. 2 Hz bzw. verd., 2-$H_2$) ; 2,78-2,96 (kompl., 5'-H u. 4B-H) ; 3,06-4,00 (komplex, 5B-H, 6'-H, 5C-H, 4'-H) ; 3,20 (br. s, 4C-H) ; 3,46 (br. s, 4A-H) ; 4,06 (dq, J = 6,5 u. 2 Hz, 5A-H) ; 4,21 (s, OH*) ; 4,42 (s, OH*) ; 4,45 (dd, J = 10 u. 1 Hz, 1B-H) ; 4,76 (dd, J = 10 u. 1 Hz, 2'-H) ; 4,79 (s, OH*) ; 4,93 (s, 1A-H) ; 5,42 (s, 1C-H) ; 6,10 (d, 1H, J = 10 Hz) ; 7,03 (d, 1H, J = 10 Hz) ; 7,19 (dt, 1H, J = 8 u. 1 Hz) ; 7,28 (dt, 1H, J = 8 u. 1 Hz) ; 7,51 (dd, 1H, J = 8 u. 1 Hz) ; 7,59 (dd, 1H, J = 8 u. 1 Hz) ; 8,10 (d, 1H, J = 1 Hz) ; 8,21 (s, 1H) ; 10,76 (s, OH oder NH*) ; 13,25 (breit, OH oder NH*) ppm

* Signale verschwinden nach $D_2O$-Austausch,
** Signale sind nur im $D_2O$-ausgetauschten Spektrum zu erkennen

$^{13}$C-NMR (200 MHz, $d_6$-DMSO, DEPT) : δ = 16,3 (o, C-6C) ; 16,9 (o, C-6A) ; 18,1 (o, C-6B) ; 18,1 (o, C-7') ; 24,0 (u, C-2A) ; 24,0 (u. C-3A) ; 29,5 (o, C-13) ; 30,6 (o) ; 65,1 (o, C-4C) ; 65,2 (o, C-4A) ; 65,2 (o, C-5C) ; 70,1 (o, C-5B) ; 70,3 (o, C-2') ; 71,4 (o, C-6') ; 74,3 (o, C-3B) ; 74,3 (o, C-5A) ; 75,2 (o, C-4B) ; 75,8 (o, C-4') ; 76,7 (o, C-5') ; 74,2 (—, C-3) ; 80,7 (—, C-4a) ; 82,4 (—, C12b) ; 91,8 (o, C-1A) ; 94,0 (o, C-1C) ; 100,9 (o, C-1B) ; 107,4 (—) ; 111,3 (—, C-7a ?) ; 115,2 (—) ; 117,3 (o) ; 120,0 (o) ; 122,7 (—) : 126,5 (—) ; 126,7 (—) ; 127,9 (—) ; 129,3 (—) ; 132,5 (o) ; 136,1 (—) ; 141,7 (—, C-9 ?) ; 142,1 (—) ; 154,9 (—, C-8 ?) ; 158,5 (—) ; 185,9 (—, C-7 ?) ; 203,9 (—, C-1) ppm
CD (Methanol) : $\lambda_{extr.}(\theta^{24} \times 10^{-4})$ = 440(+0,1) ; 395(—0,3) ; 350(+1,0) ; 320sh(—0,4) ; 278(—1,5) ; 245(+2,0) ; 226(+5,9) nm

Beispiel 8 : (Urdamycin E)

190 mg der bei der Vortrennung nach Beispiel 3 zuerst aufgefangenen roten Zone werden an Kieselgel (Chloroform/Methanol = 85 :15) und SEPHADEX LH 20 (Methanol) chromatographiert. Man erhält neben 60 mg Urdamycin B und 10 mg Urdamycin A 50 mg Urdamycin E, das durch Eintropfen einer gesättigten Aceton-Lösung in n-Hexan als roter, amorpher Feststoff ausfällt. $R_f$-Wert auf Kieselgel-Dünnschichtplatten (20 × 20 cm, 0,25 mm Schichtdicke, Laufstrecke 15 cm) von 0,61 mit Chloroform/Methanol (4 :1 Volumen) bzw. 0,45 mit Methylenchlorid/Ethanol (9 : 1 Volumen).

| | | | | |
|---|---|---|---|---|
| $C_{44}H_{58}O_{17}S$ (891,0) | Ber. | C 59,3 | H 6,6 | S 3,6 |
| | Gef. | C 59,4 | H 6,7 | S 2,3 |

IR (KBr) : 3430 ; 1733 ; 1700 sh ; 1635 ; 1610 sh $cm^{-1}$
UV (Methanol) : $\lambda_{max}(\varepsilon)$ = 460 (4500) ; 310 (6300) nm
(Methanol/HCl) : $\lambda_{max}(\varepsilon)$ = 460 (5000) ; 305 (6300) nm
(Methanol/NaOH) : $\lambda_{max}(\varepsilon)$ = 480 (5200) ; 315 (5000) ; 225 (16300) nm

$^{1}$H-NMR (200 MHz, $CD_3CN$) : δ = 0,45 (d, J = 6,5 Hz, 5C-$CH_3$) ; 1,10 (d, J = 6,5 Hz, 5A-$CH_3$) ; 1,12 (s, 3-$CH_3$) ; 1,19 (d, J = 6 Hz, 5B-$CH_3$) ; 1,33 (d, J = 6 Hz, 6'-$CH_3$) ; 1,3-2,3 (komplex, 2C-$H_2$, 2A-$H_2$, 3C-$H_2$, 3A-$H_2$, 4-$H_2$, 3'-$H_a$, 3'-$H_e$) ; 2,46 (o, J = 13,5,2 Hz 2B-$H_e$) ; 2,47 (s, S-$CH_3$) ; 2,49 u. 2,70 (dd u. d, J = 13 u. 2 bzw. 13 Hz, 2-$H_2$) ; 2,85 (dd, J = 9 u. 9 Hz, 5'-H) ; 3,08-3,60 (komplex, 4B-H, 5B-H, 4C-H, 6'-H, 3B-H, 4A-H, 5C-H) ; 3,71 (o, J = 12,9,5 Hz, 4'-H) ; 3,98 (s, OH*) ; 4,04 (s, OH*) ; 4,10 (dq, J = 6,5 u. 1,5 Hz, 5A-H) ; 4,32 (dd, J = 10 u. 1,5 Hz, 1B-H) ; 4,35 (s, OH*) ; 4,68 (dd, J = 10 u. 1 Hz, 2'-H) ; 4,76 (s, 1A-H) ; 5,06 (s, 1C-H) ; 6,41 (s, 6-H) ; 7,60 (d, J = 8 Hz, 10-H) ; 7,87 (dd, J = 8 u. 1 Hz, 11-H) ; 12,48 (breit, OH*) ppm

* Signale verschwinden nach $D_2O$-Austausch

$^{13}$C-NMR (50,3 MHz, $CD_3CN$, DEPT) : δ = 14,8 (o, $CH_3$-S) ; 17,0 (o, C-6C) ; 17,4 (o, C-6A) ; 18,4 (o, C-6B) ; 18,8 (o, C-7') ; 23,9 (u, C-3C) ; 25,2 (u, C-3A) ; 25,5 (u, C-2A) ; 26,2 (u, C-2C) ; 29,9 (o, C-13) ; 37,5 (u. C-2B) ; 40,1 (u, C-3') ; 45,8 (u, C-4) ; 54,9 (u. C-2) ; 67,1 (o, C-4C) ; 67,5 (o, C-4A) ; 68,0 (o, C-5C) ; 71,7 (o, C-5B) ; 72,0 (o, C-2') ; 72,6 (o, C-6') ; 76,1 (—, C-3) ; 76,5 (o, C-5A) ; 76,7 (o, C-3B) ; 77,1 (o, C-4B) ; 78,3 (o, C-4') ; 78,9 (o, C-5') ; 82,4 (—, C-4a) ; 84,6 (—, C-12b) ; 95,2 (o, C-1A) ; 95,6 (o, C-1C) ; 102,3 (o, C-1B) ; 105,8 (o, C-6) ; 115,1 (—, C-7a) ; 119,8 (o, C-11) ; 132,2 (—, C-11a) ; 134,6 (o, C-10) ; 134,8 (—, C-6a) ; 138,1 (—, C-9) ; 138,4 (—, C-12a) ; 158,5 (—, C-8) ; 165,2 (—, C-5) ; 183,4 (—, C-12) ; 190,1 (—, C-7) ; 203,4 (—, C-1) ppm

CD (Methanol) : $\lambda_{extr.}(\theta^{22} \times 10^{-4})$ = 495 (—0,2) ; 460 (+0,1) ; 420 (—0,4) ; 325 (+1,1) ; 253 (+2,9) ; 226 (+0,3) nm

## Beispiel 9 : (Urdamycin F)

Durch Chromatographie der gesammelten Nachzonen (100 mg) aus verschiedenen Vortrennungen nach Beispiel 3 an Kieselgel (Chloroform/Methanol = 9 :1) und SEPHADEX LH 20 (Methanol) erhält man 20 mg chromatographish einheitliches Urdamycin F, $R_f$-Wert auf Kieselgel-Dünnschichtplatten (20 × 20 cm, 0,25 mm Schichtdicke, Laufstrecke 15 cm) von 0,44 mit Chloroform/Methanol (4 :1 Volumen) bzw. 0,11 mit Methylenchlorid/Ethanol (9 :1 Volumen)

| | | | |
|---|---|---|---|
| $C_{43}H_{58}O_{18}$ (862.9) | Ber. | C 59,9 | H 6,9 |
| | Gef. | C 59,5 | H 7,3 |

IR (KBr) : 3440 ; 1727 ; 1694 ; 1666 ; 1635 ; 1608 $cm^{-1}$
UV (Methanol) : $\lambda_{max}(\varepsilon)$ = 460sh (2900) ; 432 (4300) ; 278 (5900) ; 250 (7700) ; 218 (30900) nm
(Methanol/HCl) : $\lambda_{max}(\varepsilon)$ = wie Methanol
(Methanol/NaOH) : $\lambda_{max}(\varepsilon)$ = 565 (4500) ; 277 (6600) ; 226 (25900) nm
CD (Methanol) : $\lambda_{extr.}(\theta^{26} \times 10^{-4})$ = 495 (—0,3 ; 450 (+0,2) ; 350 (—1,1) ; 310sh (—0,1) ; 275 (+1,5) ; 243 (—0,3) nm

## Beispiel 10 : Hydrolyse von Urdamycin A

a) 250 mg Urdamycin A werden in 50 ml einer Mischung aus Trifluoressigsäure, Wasser und Methanol (1 :2 :2) 10 Min. bei Raumtemperatur gerührt. Die Säure wird durch Verteilung zwischen Methylacetat und Wasser entfernt, die organische Phase wird am Rotationsverdampfer zur Trockene eingeengt, in Methanol aufgenommen und an SEPHADEX LH 20 (Methanol) chromatographiert. Man erhält 160 mg Urdamycinon A (=Aquayamycin) in chromatographish einheitlicher Form. Durch Eintropfen einer gesättigten Aceton-Lösung in n-Hexan erhält man das Hydrolyseprodukt als orangefarbenen, amorphen Feststoff, $R_f$-Wert auf Kieselgel-Dünnschichtplatten (20 × 20 cm, 0,25 mm Schichtdicke, Laufstrecke 15 cm) von 0,59 mit Chloroform/Methanol (4 :1 Volumen bzw. 0,24 mit Methylenchlorid/Ethanol (9 :1 Volumen)

b) 490 mg Urdamycin A werden in 70 ml einer Mischung aus Methanol und Wasser (5 :2) gelöst und mit 20 Tropfen konzentrierter Schwefelsäure versetzt. Es wird 30 Min. bei Raumtemperatur gerührt (im Dünnschichtchromatogramm kann dann kein Ausgangsprodukt mehr nachgewiesen werden). Es wird mit 200 ml Wasser verdünnt und mit einer gesättigten wäßrigen Bariumhydroxid-Lösung neutralisiert (pH-Meter). Es wird mit 200 ml Methanol verdünnt und das Bariumsulfat durch Zentrifugieren abgetrennt. Man entfernt die Lösemittel im Rotationsverdampfer und chromatographiert den festen Rückstand an

SEPHADEX LH 20 (Methanol) und erhält neben 220 mg Urdamycinon A 212 mg des Disaccharids Oliv-Rho, das zur weiteren Reinigung an Kieselgel (Merck KG 60-Fertigsäule, Ether/Petrolether = 1 : 1) chromatographiert wird. Man erhält 40 mg des Disaccharids als farblosen Sirup.

Urdamycinon A (= Aquayamycin)

| | | | |
|---|---|---|---|
| $C_{25}H_{26}O_{10}$ (486,5) | Ber. | C 61,7 | H 5,4 |
| | Gef. | C 61,3 | H 5,4 |

IR (KBr) : 3420 ; 1724 ; 1660 sh ; 1655 sh ; 1641 ; 1623 ; 1582 ; 1565 $cm^{-1}$
UV (Methanol) : $\lambda_{max}(\varepsilon)$ = 440 sh (4500) ; 420 (4600) ; 315 (4300) ; 230 (17700) nm
(Methanol/HCl) : $\lambda_{max}(\varepsilon)$ = wie Methanol
(Methanol/NaOH) : $\lambda_{max}(\varepsilon)$ = 565 sh (3500) ; 530 (3700) ; 385 (2600) ; 317 sh (6300) ; 281 (10500) ; 230 (16400) nm

$^1$H-NMR (200 MHz, $d_6$-DMSO) : δ = 1,11 (s, 3-$CH_3$) ; 1,20 (verd., 3'-$H_a$) ; 1,25 (d, J = 6 Hz, 6'-$CH_3$) ; 1,83 u. 2,05 (dd u. d, J = 15 u. 2 bzw. 15 Hz, 4-$H_2$) ; 2,22 (o, J = 13,5,2 Hz, 3'-$H_e$) ; 2,42 u. 2,80 (dd u. d, J = 13 u. 2 bzw. 13 Hz, 2-$H_2$) ; 2,88 (o, J = 9,9,4 Hz, 5'-H**) ; 3,34 (m, 4'-H) ; 4,78 (dd, J = 11 u. 2 Hz, 2'-H) ; 4,96 u. 5,04 (2d, J = 4 bzw. 5 Hz, 5'-OH* u. 4'-OH*) ; 5,12 (s, OH*) ; 5.26 (s, OH*) ; 6,38 (d, J = 10 Hz, 5-H) ; 6,70 (d, J = 10 Hz, 6-H) ; 7,53 (d, J = 8 Hz, 10-H) ; 7,82 (d, J = 8 Hz, 11-H) ; 12,20 (s, 8-OH*) ppm

* Signale verschwinden nach $D_2O$-Austausch,
** Signal wird nach $D_2O$-Austausch zum dd, J = 9 u. 9 Hz

CD (Methanol) : $\lambda_{extr.}(\theta^{24} \times 10^{-4})$ = 450 (+0,3) ; 395 (—0,4) ; 300 sh (+1,3) ; 267 (+1,4) ; 247 (—0,2) ; 230 sh (+1,6) nm
$\alpha_D^{20}$ (C = 1, Dioxan) : + 159°

Beispiel 11 : Hydrolyse von Urdamycin B

a) 45 mg Urdamycin B werden in 50 ml Methanol gelöst und mit 10 Tropfen konzentrierter Schwefelsäure versetzt. Nach 2 1/2 h ist alles Ausgangsmaterial umgesetzt (DC, Chloroform/Methanol = 8 :2). Die Mischung wird in Wasser gegossen und dreimal mit je 50 ml Chloroform extrahiert. Die vereinigten Chloroformphasen werden im Rotationsverdampfer zur Trockene eingedampft und an SEPHADEX LH 20 (Methanol) chromatographiert.

$R_f$-Wert auf Kieselgel-Dünnschichtplatten (20 × 20 cm, 0,25 mm Schichtdicke, Laufstrecke 15 cm) von 0,60 mit Chloroform/Methanol (4 :1 Volumen) bzw. 0,39 mit Methylenchlorid/Ethanol (9 : 1 Volumen).

b) 20 mg Urdamycin B werden in 10 ml trockenem Tetrahydrofuran gelöst und mit 3 Tropfen konzentrierter Schwefelsäure versetzt. Nach 2 Tagen wird die Mischung in Wasser gegossen, 3mal mit je 50 ml Chloroform extrahiert und zur Trockene eingeengt. Es wird an Kieselgel (Präparative Schichtplatte, 20 × 20 cm, Methylenchlorid/Methanol = 85 :15) und anschließend an SEPHADEX LH 20 (Methanol) chromatographiert. Man erhält 8 mg Urdamycinon B als gelben, amorphen Feststoff.

IR (KBr) : 3410 ; 1704 ; 1673 ; 1635 ; 1592 $cm^{-1}$
UV (Methanol) : $\lambda_{max}(\varepsilon)$ = 404 (6000) ; 286,5 (35000) nm
(Methanol/HCl) : $\lambda_{max}(\varepsilon)$ = wie Methanol
(Methanol/NaOH) : $\lambda_{max}(\varepsilon)$ = 500 (6000) ; 315 sh (7000) ; 266,5 (26000) ; 250 sh (23000) nm
CD (Methanol) : $\lambda_{extr.}(\theta^{25} \times 10^{-4})$ = 495 (—0,2) ; 410 (+0,3) ; 3.25 (+0,2) ; 264 (—2,3) nm

Beispiel 12 : Urdamycinon C

40 mg Urdamycin C werden in 20 ml einer Mischung aus Methanol und Wasser (2 : 1) gelöst und mit 20 Tropfen konz. Schwefelsäure versetzt. Nach 24 h Rühren wird auf Eis gegossen, 3 mal mit je 50 ml Chloroform extrahiert und zur Trockene eingeengt. Man chromatographiert an Kieselgel (Präparative Schichtplatte 20 × 40 cm, Methylenchlorid/Methanol = 9 : 1) und SEPHADEX LH 20 (Methanol). Der anfallende rote Farbstoff wird durch Eintropfen einer gesättigten Aceton-Lösung in n-Hexan ausgefällt. Man erhält 20 mg Urdamycinon C als roten, chromatographish einheitlichen amorphen Feststoff.

$R_f$-Wert auf Kieselgel-Dünnschichtplatten (20 × 20 cm, 0,25 mm Schichtdicke, Laufstrecke 15 cm) von 0,52 mit Chloroform/Methanol (4 :1 Volumen) bzw. 0,25 mit Methylenchlorid/Ethanol (9 : 1 Volumen).

| | | | |
|---|---|---|---|
| $C_{33}H_{30}O_{12}$ (618,6) ? | Ber. | C 64,1 | H 4,9 |
| Fp. : über 350 °C | Gef. | C 63,6 | H 5,5 |

IR (KBr) : 3420 ; 1726 ; 1708 sh ; 1640 ; 1604 $cm^{-1}$
UV (Methanol) : $\lambda_{max}(\varepsilon)$ = 513 (13200) ; 311 (15700) ; 227 (28000) nm

(Methanol/HCl) : $\lambda_{max}(\varepsilon)$ = wie Methanol
(Methanol/NaOH) : $\lambda_{max}(\varepsilon)$ = 630 sh (11500) ; 600 (12400) ; 412 (5000) ; 323 (9600) ; 240 (30400) nm

$^1$H-NMR (200 MHz, $d_6$-Aceton) : δ = 1,18 (s, 3-$CH_3$) ; 1,21 (d, J = 6 Hz, 6'-$CH_3$) ; 1,23 (o, J = 12, 11 u. 11 Hz, 3'-$H_a$) ; 2,1 (dd, J = 12 u. 12 Hz 4-$H_2$) ; 2,41 (o, J = 12,5,2 Hz, 3'-$H_e$) ; 2,78 (dd, J = 13 u. 2 Hz, 2-$H_e$) ; 2,96 (dd, J = 10 u. 9 Hz, 5'-H) ; 3,23 (d, J = 13 Hz, 2-$H_a$) ; 3,41 (dq, J = 9 u. 6 Hz, 6'-H) ; 3,70 (o, J = 11,9,5 Hz, 4'-H) ; 3,87 (s, OH*) ; 4,14 (breit, 1-2 OH*) ; 4,70 (dd, J = 10 u. 1,5 Hz, 2'H) ; 4,74 (s, OH*) ; 4,90 (s, OH*) ; 5,26 (s, OH*) ; 6,14 (d, 1H, J = 10 Hz) ; 6,99 (d, 1H, J = 10 Hz) ; 7,03 (d, 2H, J = 8 Hz) ; 7,43 (d, 2H, J = 8 Hz) ; 7,87 (d, 1H, J = 2 Hz) ; 9,00 (breit, OH*) ; 13,16 (breit, OH*) ppm

* Signale verschwinden nach Austausch mit $CD_3OD$

C-NMR (50,3 MHz, $d_6$-DMSO, APT) : δ = 18,1 (o, C-7') ; 29,6 (o, C-13) ; 30,6 (o) ; 43,7 (u, C-4) ; 52,0 (u, C-2) ; 70,6 (o, C-2') ; 71,4 (o, C-6') ; 74,9 (u, C-3) ; 75,8 (o, C-4') ; 76,7 (o, C-5') ; 78,3 (u, C-4a) ; 80,1 (u, C-12b) ; 111,0 (u, C-7a ?) ; 114,9 (o) ; 114,9 (o) ; 115,3 (u) ; 117,3 (o) ; 122,1 (u) ; 223,8 (u) ; 129,0 (u) ; 131,4 (u) ; 132,5 (u) ; 133,2 (o) ; 133,5 (o) ; 138,2 (o) ; 140,7 (u, C-9 ?) ; 143,7 (u) ; 154,9 (u, C-8 ?) ; 158,7 (u) ; 159,0 (u) ; 186,1 (u) ppm

(50,3 MHz, $CD_3OD$) : δ = 18,5 (C-7') ; 30,2 (C-13) ; 30,7 ; 40,9 (C-3') ; 45,0 (C-4) ; 53,1 (C-2) ; 72,3 (C-2') ; 73,3 (C-6') ; 76,4 (C-3) ; 77,4 (C-4') ; 78,6 (C-5') ; 79,7 (C-4a) ; 81,6 (C-12b) ; ca. 113 (C-7a ?) ; 116,1 ; 116,1 ; ca. 117 ; 119,5 ; 123,9 ; 125,8 ; 129,6 ; 133,1 ; 134,5 ; 134,6 ; 134,7 ; 138,3 ; ca. 142 (C-9 ?) ; 145,4 ; ca. 157 (C-8 ?) ; 160,6 ; 160,8 ; ca. 188 (C-7 ?) ; ca. 208 (C-1) ppm

CD (Methanol) : $\lambda_{extr.}(\theta^{24} \times 10^{-4})$ = 520 (+0,6) ; 420 (0) ; 340 (+1,0) ; 314 (—0,6) ; 295 (0) ; 275 (—0,6) ; 246 (+0,9) ; 239 (+0,7) ; 221 (+3,8) nm

### Beispiel 13 : Urdamycinon E

30 mg Urdamycin E werden in 20 ml einer Mischung aus Methanol und Wasser (2 : 1) gelöst und mit 10 Tropfen konz. Schwefelsäure versetzt. Nach 1 h wird die Mischung in Wasser gegossen, 3mal mit je 30 ml Chloroform extrahiert und im Rotationsverdampfer zur Trockene eingeengt. Den festen Rückstand chromatographiert man an Kieselgel (Präparative Schichtplatte, 20 × 20 cm, Methylenchlorid/Ethanol = 9 : 1) und SEPHADEX LH 20 (Methanol). Man erhält 18 mg Urdamycinon E als roten, amorphen Feststoff. $R_f$-Wert auf Kieselgel-Dünnschichtplatten (20 × 20 cm, 0,25 mm Schichtdicke, Laufstrecke 15 cm) von 0,61 mit Chloroform/Methanol (4 : 1 Volumen bzw. 0,45 mit Methylenchlorid/Ethanol (9 : 1 Volumen).

$C_{26}H_{28}O_{10}S$ (532,6)
IR (KBr) : 3420 ; 1721 ; 1679 ; 1634 ; 1605 sh ; 1580 sh $cm^1$
UV (Methanol) : $\lambda_{max}(\varepsilon)$ = 480 (6700) ; 282 (12800) ;
(Methanol/HCl) : $\lambda_{max}(\varepsilon)$ = wie Methanol
(Methanol/NaOH) : $\lambda_{max}(\varepsilon)$ = 480 sh (7300) ; 350 sh (4500) ; 275 (11400) ; 225 (16800) ;
CD (Methanol) : $\lambda_{extr.}$ $(\theta^{25} \times 10^{-4})$ = 500 (—0,5) ; 360 (—0,2) ; 315 (+0,03) ; 292 (+1,1) ; 276 (+0,6) ; 250 (+2,6) nm

### Beispiel 14 : Urdamycin A-pentaacetat

60 mg Urdamycin A werden in 15 ml einer Mischung aus Acetanhydrid und Pyridin (2 :1) gelöst und 7 h bei Raumtemperatur gerührt. Die Mischung wird auf Eis gegossen, 3 mal mit je 50 ml Chloroform extrahiert und am Rotationsverdampfer zur Trockene eingedampft. Pyridin-Reste entfernt man durch mehrmaliges Aufnehmen und Eindampfen mit Toluol. Es wird an Kieselgel (Präparative Schichtplatte, 20 × 40 cm, Chloroform/Methanol = 98 :2) chromatographiert und das gelbe Hauptprodukt n SEPHADEX LH 20 (Säule 50 × 2,5 cm, Methanol) nochmals chromatographiert. Man erhält 23 mg Urdamycinpentaacetat der Formel VII als gelben Feststoff, $R_f$-Wert auf Kieselgel-Dünnschichtplatten (20 × 20 cm, 0,25 mm Schichtdicke, Laufstrecke 15 cm) von 0,83 mit Chloroform/Methanol (4 : 1 Volumen) bzw. 0,91 mit Methylenchlorid/Ethanol (9 : 1 Volumen).

| | | | |
|---|---|---|---|
| $C_{53}H_{66}O_{22}$ (1055,1) | Ber. | C 60,3 | H 6,3 |
| Fp. : 185 °C | Gef. | C 60,4 | H 6,4 |

IR (KBr) : 3470 ; 1783 ; 1741 ; 1667 ; 1632 ; 1599 $cm^{-1}$
UV (Methanol) : $\lambda_{max}(\varepsilon)$ = 355 (4300) ; 313 (4900) ; 257 (15900) nm
(Methanol/HCl) : $\lambda_{max}(\varepsilon)$ = wie Methanol
(Methanol/NaOH) : $\lambda_{max}(\varepsilon)$ = 395 sh (2600) ; 375 sh (3500) ; 327 (7400) ; 253 (10400) nm

$^1$H-NMR (200 MHz, $CDCl_3$) : δ = 0,50 (d, J = 6,5 Hz, 5C-$CH_3$) ; 1,18 (d, J = 6,5 Hz, 5A-$CH_3$) ; 1,19 (d, J = 6 Hz, 5B-$CH_3$) ; 1,25 (s, 3-$CH_3$) ; 1,27 (d, J = 6 Hz, 6'-$CH_3$) ; 1,2-2,2 (komplex, 13H) ; 2,00, 2,04, 2,06 u. 2,10 (4s, 5', 3B-, 4B- und 4C-OAc) ; 2,36 (o, J = 13,5, 2 Hz, 2B-$H_e$) ; 2,48 (s, 8-OAc) ; 2,52 u. 2,80 (d. u. dd., J = 13 bzw. 13 u. 2 Hz, 2-$H_2$) ; 3,44 (dq, J = 9 u. 6 Hz, 5B-H) ; 3,48 (s, breit, 4A-H) ; 3,57 (s, OH*) ; ca. 3,6 (verd., 6'-H) ; 3,68 (dq, 6,5 u. 2 Hz, 5C-H) ; 3,87 (dq, J = 6,5 u. 2 Hz, 5A-H) ; 4,04 (m, 4'-H) ; 4,06 (s, OH*) ; 4,56 (dd, J = 10 u. 2 Hz, 1B-H) ; 4,67 (o, J = 3x ca. 2 Hz, 4C-H) ; 4,6-4,8 (verd., 2'-H) ; 4,74 u. 4,82 (2 dd, J = je 9 u. 9 Hz, 5'- u. 4B-H) ; 4,90-5,02 (kompl., 1A-H u. 3B-H) ; 5,46 (d, J = 2 Hz, 1C-H) ; 6,41 (d, J = 10 Hz, 5-H) ; 6,85 (d, J = 10 Hz, 6-H) ; 8,02 (d, J = 8 Hz, 10-H) ; 8,13 (d, J = 8 Hz, 11-H) ppm

* Signale verschwinden nach $CD_3OD$-Austausch

CD (Methanol) : $\lambda_{extr.}(\theta^{22} \times 10^{-4})$ = 340 (—1,4) ; 280 (+4,3) ; 250 (+3,2) ; 226 (+5,8) nm

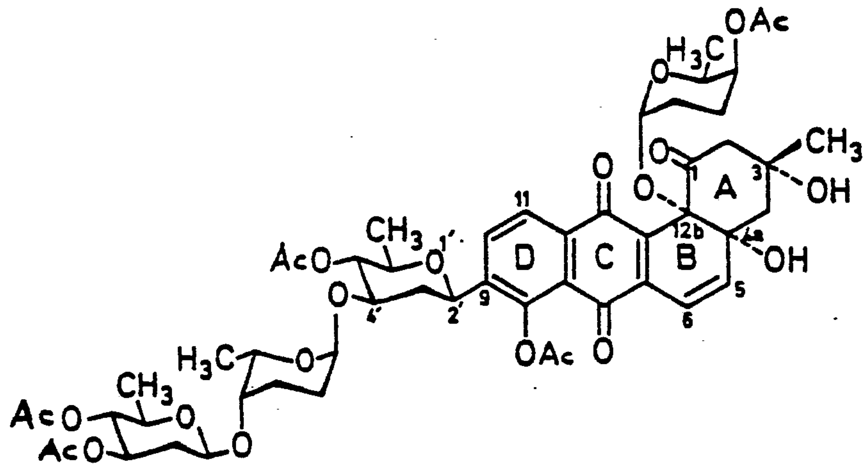

(VIII)

Beispiel 15

3-(2',3',7'-Tridesoxy-β-D-arabino-hexopyranos-2'-yl)-4-hydroxy-7-methoxy-9-methyl-naphthacen-5,12-chinon

Bei der Hydrolyse von Urdamycin B in Methanol/Schwefelsäure gemäß Beispiel 11 kann man aus dem Eluat bei der Chromatographie als Nebenprodukt das Naphthacen-chinon der Formel IX isolieren : Durch Eintropfen einer gesättigten Methylenchlorid-Lösung in n-Pentan erhält man 12 mg eines chromatographisch einheitlichen, gelben, amorphen Feststoffs.

$C_{26}H_{24}O_7$ (448,5)

IR (KBr) : 3440 ; 1670 ; 1627 $cm^{-1}$
UV (Methanol) : $\lambda_{max}(\varepsilon)$ = 410 (7300) ; 305 (31500) ; 243 sh (29200) ; 225 (42500) nm
(Methanol/HCl) : $\lambda_{max}(\varepsilon)$ = wie Methanol
(Methanol/NaOH) : $\lambda_{max}(\varepsilon)$ = 500 (6400) ; 293 (31700) ; 243 (30100) ; 219 (33700) nm
$^1$H-NMR (200 MHz, $d_6$-DMSO) : δ = 1,26 (d, J = 6 Hz, 6'-$CH_3$) ; 1,29 (o, J = 3 × 12 Hz, 3'-$H_a$) ; 2,24 (o, J = 12,5, 2 Hz, 3'-$H_e$) ; 2,50 (s, 9-$CH_3$) ; 2,89 (dd, J = 9 u. 9 Hz, 5'-H) ; 3,34 (dq, J = 9 u. 6 Hz, 6'-H) ; 3,54 (o, J = 12,9, 5 Hz, 4'-H) ; 3,88 (s, 7-$OCH_3$) ; 4,79 (dd, J = 9 u. 1,5 Hz, 2'-H) ; 4,97 u. 5,05 (2d, J = je 4 Hz, 4'-OH* u. 5'-OH*) ; 7,13 (s, 8-H) ; 7,24 (s, 10-H) ; 7,33 (d, J = 8 Hz, 2-H) ; 7,43 (d, J = 8 Hz, 1-H) ; 8,13 (s, 2H, 6-H u. 11-H) ; 12,42 (breit, 4-OH*) ; ppm

* Signale verschwinden nach $D_2O$-Austausch

CD (Methanol) : $\lambda_{extr.}(\theta^{24} \times 10^{-4})$= 390sh (+ 0,3), 335sh (+ 0,2) ; 290 (— 0,4) ; 245sh (+ 0,4) nm

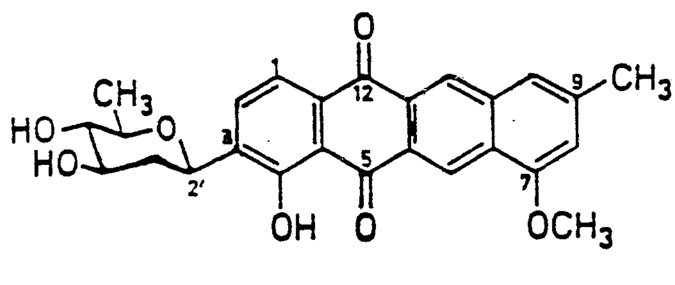

(IX)

Beispiel 16

3-(2',3',7'-Tridesoxy-β-D-arabino-hexopyranos-2'-yl)-4-hydroxy-7-ethoxy-9-methyl-naphthacen-5,12-chinon

Bei der Hydrolyse von Urdamycin B gemäß Beispiel 11, jedoch unter Einsatz von Ethanol anstelle von Methanol, erhält man entsprechend Beispiel 15 aus 20 mg Urdamycin B 5 mg des Naphthacen-chinons der Formel X.

$C_{27}H_{26}O_7$ (462,5)

$^1$H-NMR (80 MHz, $d_6$-Aceton) : δ = 1,35 (d, J = 6 Hz, 6'-$CH_3$) ; 1,35 (o, J = 3 × 12 Hz, 3'-$H_a$) ; 1,43 (t, J = 6,5 Hz, Ethoxy-$CH_3$) ; 2,35 (o, J = 12,5, 2 Hz, 3'-$H_e$) ; 2,50 (s, 9-$CH_3$) ; 3,05 (dd, JO 9 u. 9 Hz, 5'-H) ; 3,40 (dq, J = 6 u. 9 Hz, 6'-H) ; 3,75 (o, J = 12,9, 5 Hz, 4'-H) ; 4,18 (q, J = 6,5 Hz, Ethoxy-$OCH_2$) ; 4,87 (dd, J = 10 u. 2 Hz, 2'-H) ; 7,02 (s, 8-H) ; 7,32 (s, 10-H) ; 7,47 (d, J = 8 Hz, 2-H) ; 7,85 (d, J = 8 Hz, 1-H) ; 8,02 u. 8,06 (2s, 6-H u. 11-H) ppm

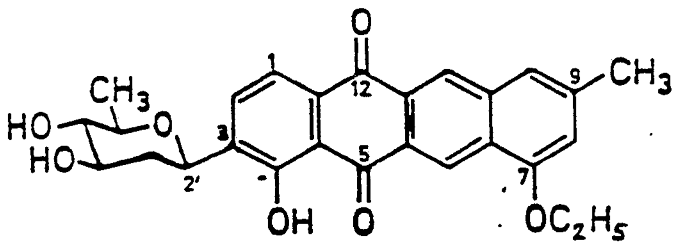

(X)

**Patentansprüche** (für die Vertragsstaaten : BE, CH, DE, FR, GB, IT, LI, LU, NL, SE)

1. Urdamycin A der Formel I

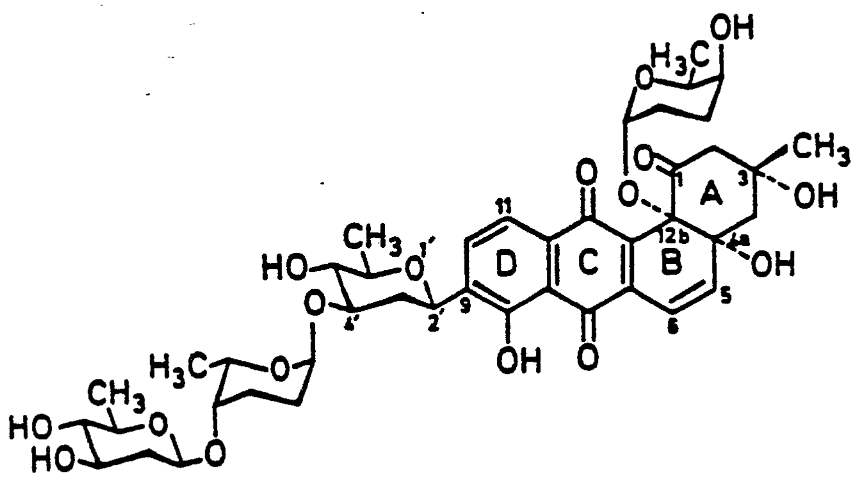

(I)

2. Urdamycin B der Formel II

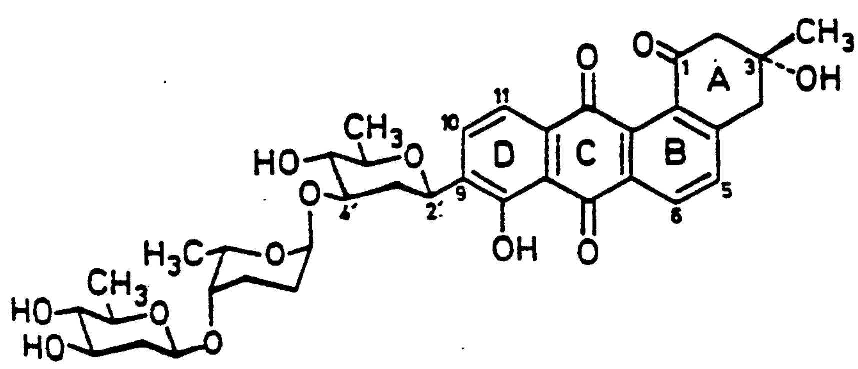

(II)

3. Urdamycin E der Formel III

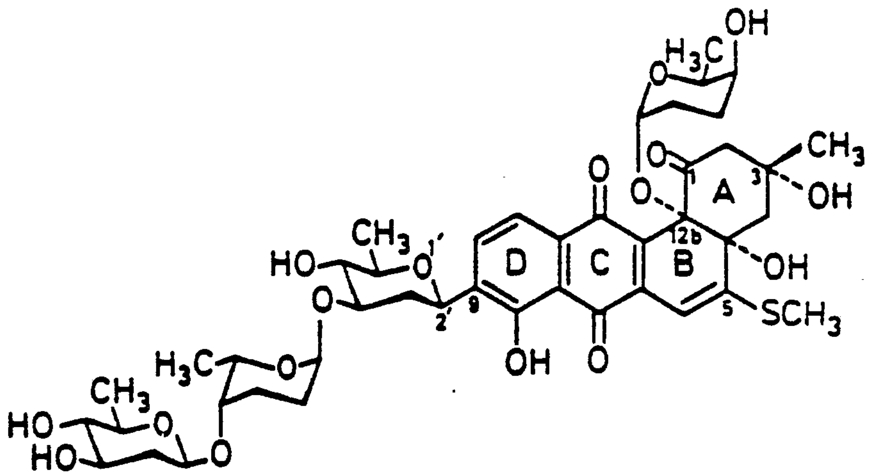

(III)

4. Urdamycin F der Formel IV

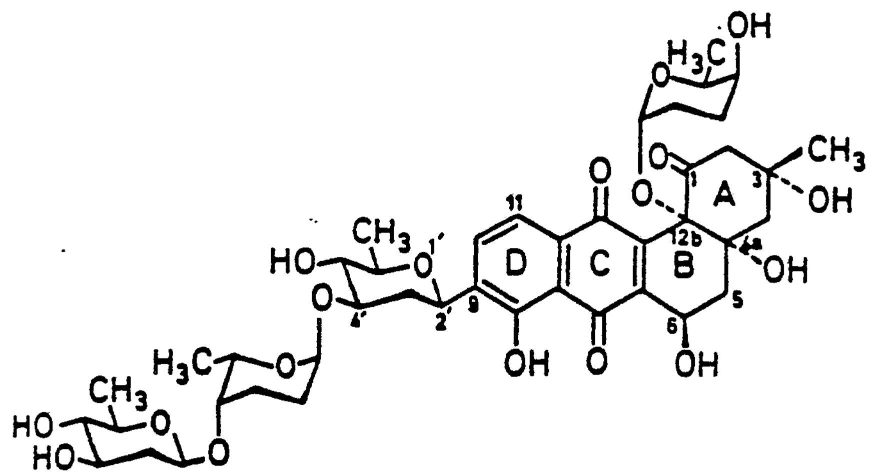

(IV)

5. Urdamycin C, gekennzeichnet durch einen Schmelzpunkt von 200 °C (unter Zersetzung) ; einen C-Gehalt von etwa 63,4 %, einen H-Gehalt von etwa 6,7 % ; IR-Banden (in KBr) bei 3420, 1732, 1705 (sh), 1640 und 1605 $cm^{-1}$ ; UV-Banden (in Methanol) $\lambda_{max}(\varepsilon)$ 510 (12600), 410 (6300, sh) 308 (14800), 237 (23600, sh) und 226 nm (25300) bzw. (in Methanol/HCl) $\lambda_{max}(\varepsilon)$ 510 (12600), 310 (15400) und 226 nm (26900) bzw. (in Methanol/NaOH) $\lambda_{max}(\varepsilon)$ 625 (10400, sh), 600 (12100), 414 (5500), 326 (12000) und 238 nm (30000) ; ein Zirkular-Dichroismus-Spektrum in Methanol $\lambda_{extr.}(\theta^{21}\cdot10^{-4}$ = 500 (— 0,5), 336 (+ 1,7), 283 (— 1,2), 248 (+ 2,9), 236 (+ 2,2) und 223 nm (+ 4,8) ; sowie einen $R_f$-Wert auf Kieselgel-Dünnschichtplatten (20·20 cm, 0,25 mm Schichtdicke, Laufstrecke 15 cm) von 0,51 mit Chloroform/Methanol (4 :1 Volumen) bzw. 0,15 mit Methylenchlorid/Ethanol (9 :1 Volumen).

6. Urdamycin D, gekennzeichnet durch einen Schmelzpunkt von 220 °C (unter Zersetzung) ; einen C-Gehalt von etwa 64,1 %, einen H-Gehalt von etwa 6,5 % une einen N-Gehalt von etwa 1,5 % ; IR-Banden (in KBr) bei 3420, 1735, 1710 (sh), 1690, 1668 und 1595 $cm^{-1}$ ; UV-Banden (in Methanol mit und ohne HCl $\lambda_{max}(\varepsilon)$ 575 (11200) und 328 nm (11600) bzw. (in Methanol/NaOH) 635 (7200, sh), 600 (8500), 412 (4200), 324 (7500), 270 (17600, sh) und 222 nm (32400) ; einen Zirkular-Dichroismus-Spektrum in Methanol $\lambda_{extr.}(\theta^{24}\cdot10^{-4})$ = 440 (+ 0,1), 395 (— 0,3), 350 (+ 1,0), 320 sh (— 0,4), 278 (— 1,5), 245 (+ 2,0) und 226 nm (+ 5,9) ; sowie einen $R_f$-Wert auf Kieselgel-Dünnschichtplatten (20 .20 cm, 0,25 mm Schichtdicke, Laufstrecke 15 cm) von 0,52 mit Chloroform/Methanol (4 :1 Volumen) bzw. 0,17 mit Methylenchlorid/Ethanol (9 :1 Volumen).

7. Urdamycine A bis F nach den Ansprüchen 1 bis 6 erhältlich durch aerobe Fermentation von Streptomyces fradiae DSM 3093 und Isolierung aus dem Kulturmedium.

8. Urdamycine A bis F nach Anspruch 1, dadurch gekennzeichnet, daß die Isolierung mit Hilfe

a) einer Extraktion des Kulturmediums mit einem wenig oder nicht mit Wasser mischbaren organischen Lösemittel,

b) Entfernung stark lipophiler Bestandteile und

c) Chromatographie an Kieselgel unter Verwendung einer Mischung aus einem niederen Chlorkohlenwasserstoff als Hauptbestandteil und einem niederen Alkanol als untergeordnetem Bestandteil als Laufmittel durchgeführt wird, wobei die Urdamycine in der Reihenfolge B, E, A, D, C und F isoliert werden.

9. Verfahren zur Herstellung von antibiotisch wirksamen Verbindungen durch aerobe Fermentation von Streptomyceten, dadurch gekennzeichnet, daß man den Stamm S. fradiae DSM 3093 fermentiert, aus dem Kulturmedium die antibiotisch wirksamen Komponenten extrahiert und gegebenenfalls auftrennt.

10. Streptomyces fradiae DSM 3093.

11. Verwendung der Urdamycine nach Anspruch 1 bis 8 bzw. der nach Anspruch 9 erhaltenen Verbindungen zur Herstellung von Heilmitteln.

12. Verwendung von Urdamycin A gemäß Anspruch 3 zur Herstellung von Aquayamycin.

13. Verwendung der Urdamycine B bis F gemäß den Ansprüchen 4 bis 8 zur Herstellung der Aglykone.

14. Verhfahren zur Herstellung von Urdamycinon B bis F, dadurch gekennzeichnet, daß man aus Urdamycin B bis F gemäß den Ansprüchen 4 bis 8 die O-glykosidisch gebundenen Zucker sauer abspaltet.

15. Urdamycinon B der Formel V

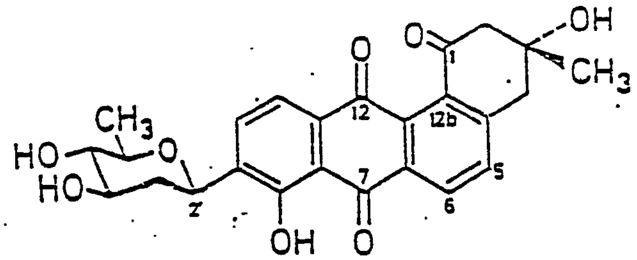

(V)

16. Urdamycinon E der Formel VI

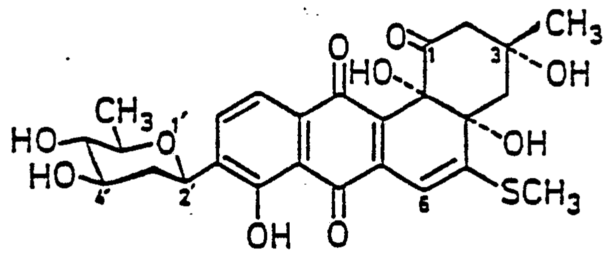

(VI)

17. Urdamycinon F der Formel VII

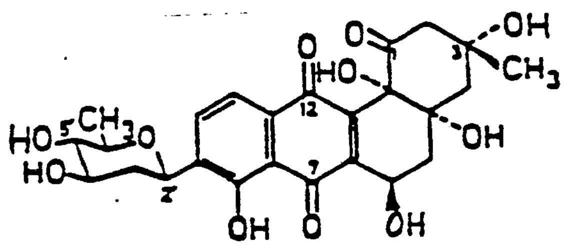

(VII)

18. Urdamycinon C, erhältlich aus Urdamycin C gemäß Anspruch 7 durch saure Abspaltung der O-glykosidisch gebundenen Zuckermoleküle.

19. Urdamycinon D, erhältlich aus Urdamycin D gemäß Anspruch 8 durch saure Abspaltung der O-glykosidisch gebundenen Zuckermoleküle.

20. Urdamycinon C, gekennzeichnet durch einen Schmelzpunkt über 350 °C; einen C-Gehalt von etwa 63,6 % und einen H-Gehalt von etwa 5,5 %; IR-Banden (in KBr) bei 3420, 1726, 1708 (sh), 1640 und 1604 $cm^{-1}$; UV-Banden (in Methanol, mit und ohne HCl) $\lambda_{max}(\varepsilon)$ 513 (13200), 311 (15700) und 227 nm (28000); bzw. (in Methanol/NaOH) 630 (11500, sh) 600 (12400), 412 (5000), 323 (9600) und 240 nm (30400); ein Zirkular-Dichroismus-Spektrum in Methanol $\lambda_{extr.}(\theta^{24} \cdot 10^{-4})$ = 520 (+ 0,6), 420 (0), 340 (+ 1,0), 314 (— 0,6), 295 (0), 275 (— 0,6), 246 (+ 0,9), 239 (+ 0,7) und 221 nm (+ 3,8); sowie einen $R_f$-Wert auf Kieselgel-Dünnschichtplatten (20·20 cm, 0,25 mm Schichtdicke, Laufstrecke 15 cm) von 0,52 mit Chloroform/Methanol (4 :1 Volumen) bzw. 0,15 mit Methylenchlorid/Ethanol (9 :1 Volumen).

21. Verwendung von Urdamycinon B bis F gemäß den Ansprüchen 15 bis 20 zur Herstellung von Heilmitteln.

22. Urdamycin A bis F gemäß den Ansprüchen 3 bis 8 und Urdamycinon B bis F gemäß den Ansprüchen 15 bis 20 zur Anwendung in Heilverfahren.

**Patentansprüche** (für den Vertragsstaat AT)

1. Verfahren zur Herstellung antibiotisch wirksamer Verbindungen durch aerobe Fermentation von Streptomyceten, dadurch gekennzeichnet, daß man
   a) den Stamm Streptomyces fradiae DSM 3093 fermentiert und
   b) die Urdamycine aus dem Kulturmedium extrahiert.
2. Verfahren zur Herstellung antibiotisch wirksamer Verbindungen durch aerobe Fermentation von Streptomyceten, dadurch gekennzeichnet, daß man
   a) den Stamm Streptomyces fradiae DSM 3093 fermentiert,
   b) die Urdamycine aus dem Kulturmedium extrahiert und
   c) die Urdamycine auftrennt.
3. Verfahren nach Anspruch 2, dadurch gekennzeichnet, daß die Extraktion des Kulturmediums mit einem organischen Lösemittel durchgeführt wird, das mit Wasser wenig oder nicht mischbar ist, stark lipophile Bestandteile aus dem Extrakt entfernt werden und durch Chromatographie an Kieselgel mit einem Elutionsmittel, das aus einem niederen Chlorkohlenwasserstoff als Hauptbestandteil und einem niederen Alkanol als untergeordnetem Bestandteil besteht, die Urdamycine in der Reihenfolge Urdamycin B der Formel II,

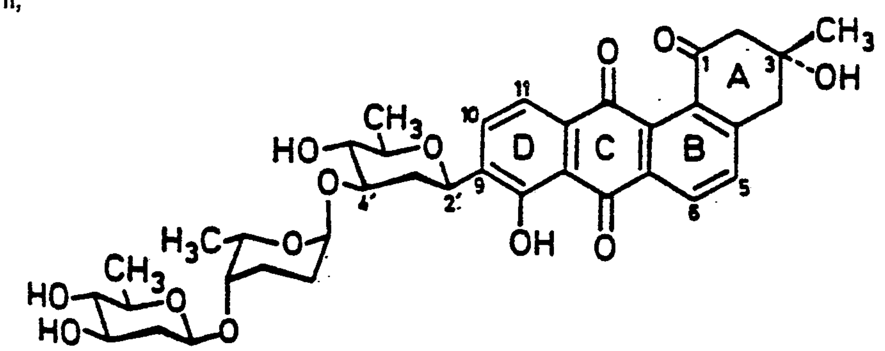

(II)

Urdamycin E der Formel III,

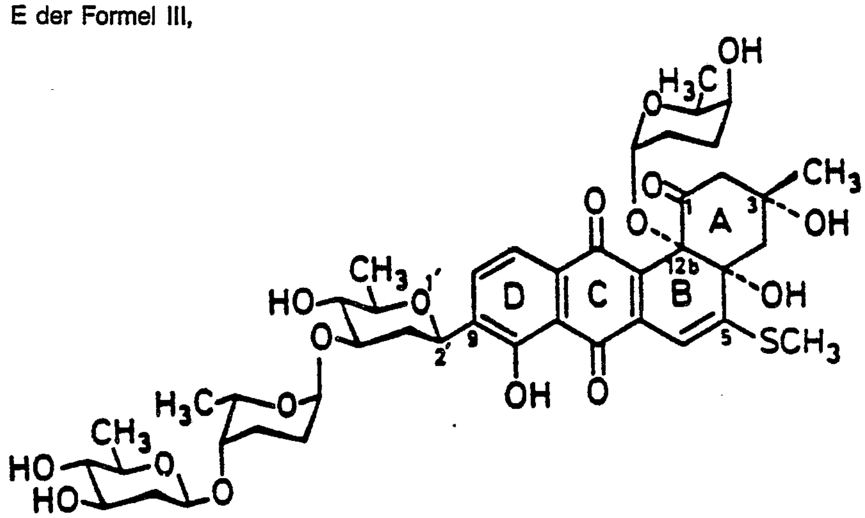

(III)

Urdamycin A der Formel I,

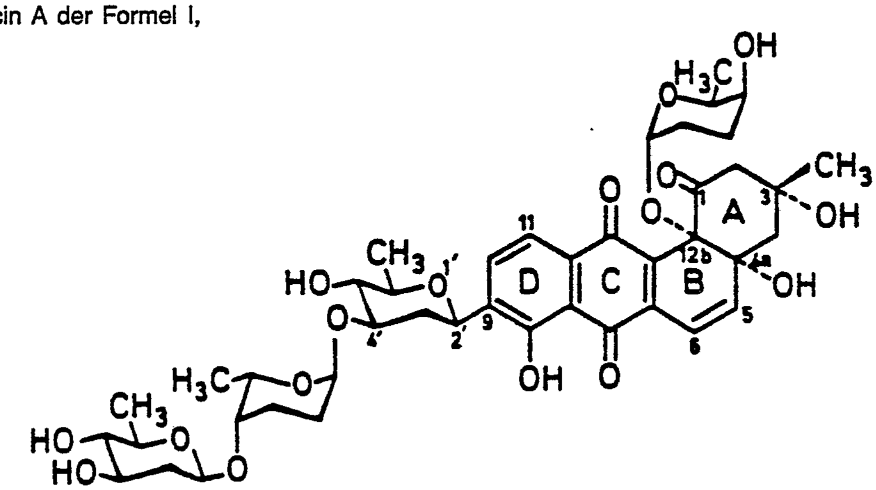

(I)

Urdamycin D, gekennzeichnet durch einen Schmelzpunkt von 220 °C (unter Zersetzung) ; einem C-Gehalt von etwa 64,1 %, einen H-Gehalt von etwa 6,5 % und einen N-Gehalt von etwa 1,5 % ; IR-Banden (in KBr) bei 3420, 1735, 1710 (sh), 1690, 1668 und 1595 $cm^{-1}$ ; UV-Banden (in Methanol mit und ohne HCl $\lambda_{max}(\varepsilon)$ 575 (11200) und 328 nm (11600) bzw. (in Methanol/NaOH) 635 (7200, sh), 600 (8500), 412 (4200), 324 (7500), 270 (17600, sh) und 222 nm (32400) ; ein Zirkular-Dichroismus-Spektrum in Methanol $\lambda_{extr.}(\theta^{24} \cdot 10^{-4})$ = 440 (+ 0,1), 395 (— 0,3), 350 (+ 1,0), 320 sh (— 0,4), 278 (— 1,5), 245 (+ 2,0) und 226 nm (+ 5,9) ; sowie einen $R_f$-Wert auf Kieselgel-Dünnschichtplatten (20·20 cm, 0,25 mm Schichtdicke, Laufstrecke 15 cm) von 0,52 mit Chloroform/Methanol (4 :1 Volumen) bzw. 0,17 mit Methylenchlorid/Ethanol (9 :1 Volumen),

Urdamycin C, gekennzeichnet durch eine Schmelzpunkt von 200 °C (unter Zersetzung) ; eine C-Gehalt von etwa 63,4 %, einen H-Gehalt von etwa 6,7 % ; IR-Banden (in KBr) bei 3420, 1732, 1705 (sh), 1640 und 1605 $cm^{-1}$ ; UV-Banden (in Methanol) $\lambda_{max}(\varepsilon)$ 510 (12600), 410 (6300, sh) 308 (14800), 237 (23600, sh) und 226 nm (25300) bzw. (in Methanol/HCl) $\lambda_{max}(\varepsilon)$ 510 (12600), 310 (15400) und 226 nm (26900) bzw. (in Methanol/NaOH) $\lambda_{max}(\varepsilon)$ 625 (10400, sh), 600 (12100), 414 (5500), 326 (12000) und 238 nm (30000) ; ein Zirkular-Dichroismus-Spektrum in Methanol $\varepsilon_{extr.}(\theta^{21} \cdot 10^{-4}$ = 500 (— 0,5), 336 (+ 1,7), 283 (— 1,2), 248 (+ 2,9), 236 (+ 2,2) und 223 nm (+ 4,8) ; sowie einen $R_f$-Wert auf Kieselgel-Dünnschichtplatten (20·20 cm, 0,25 mm Schichtdicke, Laufstrecke 15 cm) von 0,51 mit Chloroform/Methanol (4 :1 Volumen) bzw. 0,15 mit Methylenchlorid/Ethanol (9 :1 Volumen), und

Urdamycin F der Formel IV

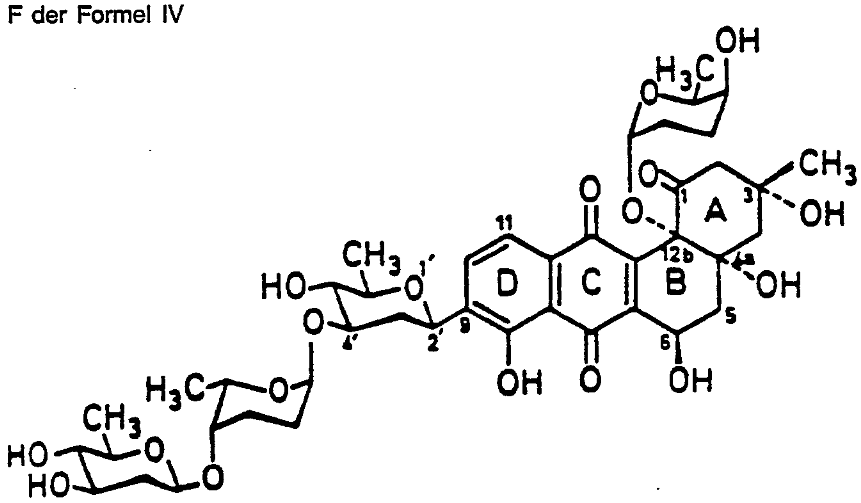

(IV)

eluiert werden.

4. Verfahren zur Herstellung von Urdamycinon B bis F, dadurch gekennzeichnet, daß man aus Urdamycin B bis F gemäß Anspruch 3 die O-glykosidisch gebundenen Zucker sauer abspaltet und

Urdamycinon B der Formel V,

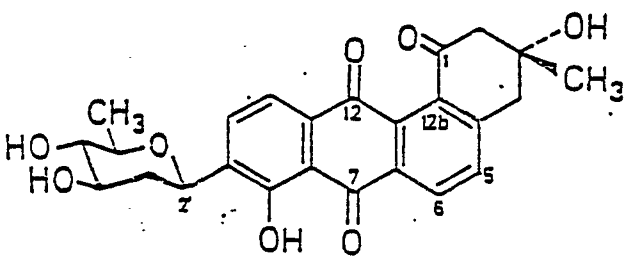

(V)

Urdamycinon E der Formel VI,

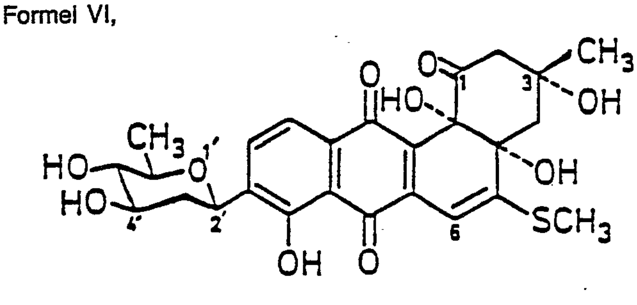

(VI)

Urdamycinon F der Formel VII,

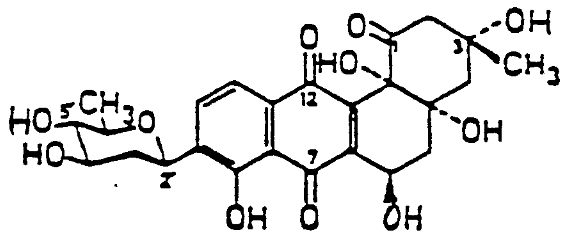

(VII)

Urdamycinon C, gekennzeichnet durch einen Schmelzpunkt über 350 °C; einen C-Gehalt von etwa 63,6 % und einen H-Gehalt von etwa 5,5 %; IR-Banden (in KBr) bei 3420, 1726 1708 (sh), 1640 und 1604 $cm^{-1}$; UV-Banden (in Methanol, mit und ohne HCl) $\lambda_{max}(\varepsilon)$ 513 (13200), 311 (15700) und 227 nm (28000); bzw. (in Methanol/NaOH) 630 (11500, sh) 600 (12400), 412 (5000), 323 (9600) und 240 nm (30400); ein Zirkular Dichroismus-Spektrum in Methanol $\lambda_{extr.}(\theta^{24}\cdot 10^{-4})$ = 520 (+ 0,6), 420 (0), 340 (+ 1,0), 314 (— 0,6), 295 (0), 275 (— 0,6), 246 (+ 0,9), 239 (+ 0,7) und 221 nm (+ 3,8); sowie einen $R_f$-Wert auf Kieselgel-Dünnschichtplatten (20·20 cm, 0,25 mm Schichtdicke, Laufstrecke 15 cm) von 0,52 mit Chloroform/Methanol (4 :1 Volumen) bzw. 0,15 mit Methylenchlorid/Ethanol (9 :1 Volumen) und

Urdamycinon D erhält.

5. Verfahren zur Herstellung von Aquayamycin, dadurch gekennzeichnet, daß man als Ausgangsprodukt Urdamycin A gemäß Anspruch 3 einsetzt.

**Claims** (for the Contracting States : BE, CH, DE, FR, GB, IT, LI, LU, NL, SE)

1. Urdamycin A of the formula I

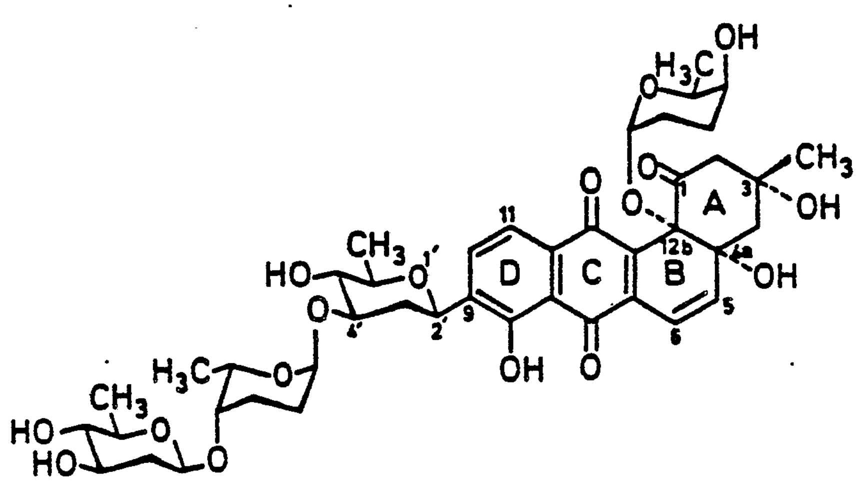

(I)

2. Urdamycin B of the formula II

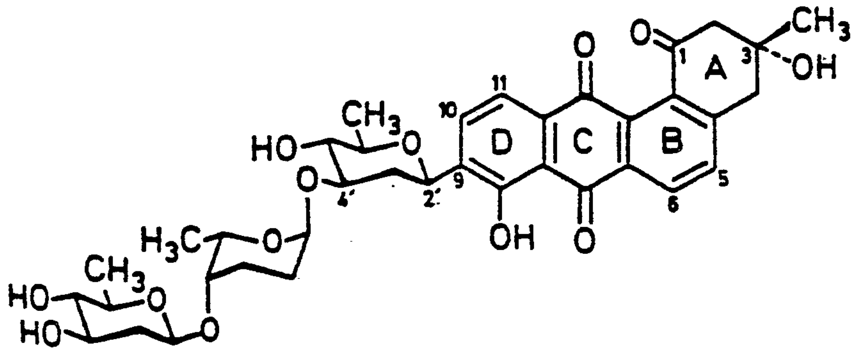

(II)

3. Urdamycin E of the formula III

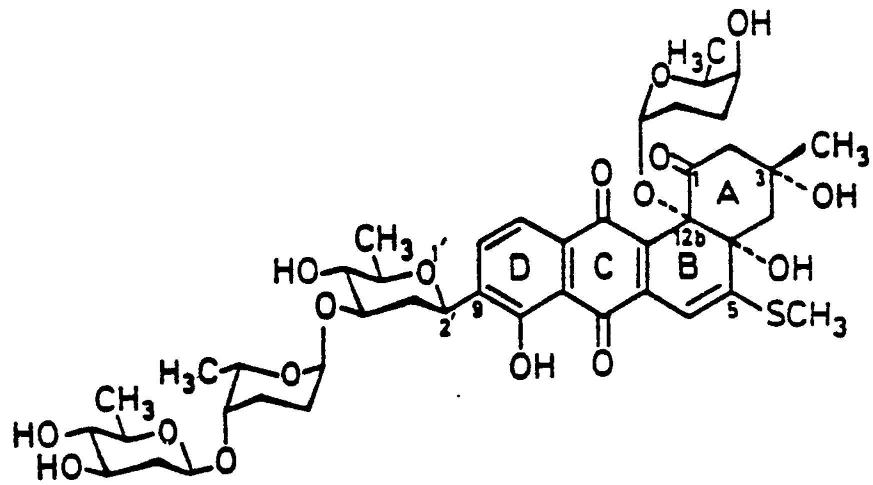

(III)

4. Urdamycin F of the formula IV

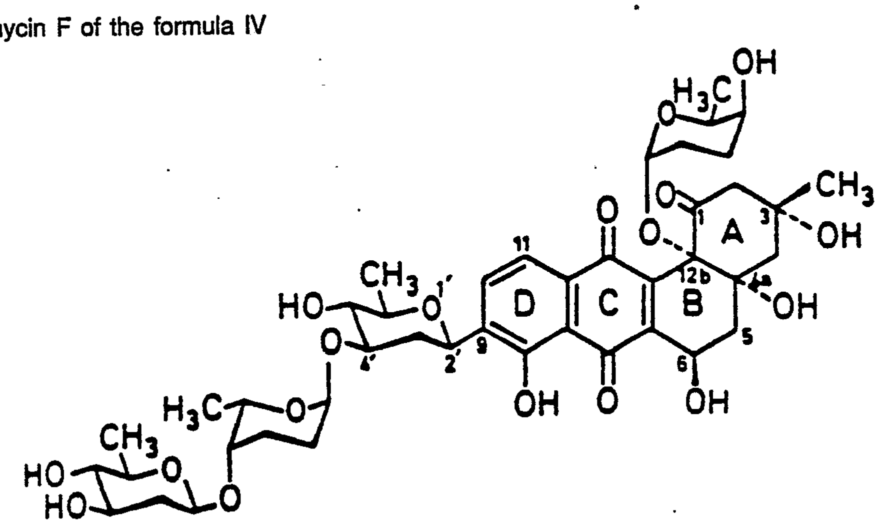

(IV)

5. Urdamycin C, which has a melting point of 200 °C (with decomposition) ; a C content of about 63.4 %, an H content of about 6.7 %, IR bands (in KBr) at 3420, 1732, 1705 (sh), 1640 and 1605 $cm^{-1}$ ; UV bands (in methanol) $\lambda_{max}(\varepsilon)$ 510 (12600), 410 (6300, sh), 308 (14800), 237 (23600, sh) and 226 nm (25300) or (in methanol/HCl) $\lambda_{max}(\varepsilon)$ 510 (12600), 310 (15400) and 226 nm (26900) or (in methanol/NaOH) $\lambda_{max}(\varepsilon)$ 625 (10400, sh), 600 (12100), 414 (5500), 326 (12000) and 238 nm (30000) ; a circular dichroism spectrum in methanol $\lambda_{extr.}(\theta^{21} \times 10^{-4}$ = 500 (— 0.5), 336 (+ 1.7), 283 (— 1.2), 248 (+ 2.9), 236 (+ 2.2) and 223 nm (+ 4.8) ; and an $R_f$ value on silica gel thin layer plates (20 × 20 cm, 0.25 mm layer thickness, development distance 15 cm) of 0.51 with chloroform/methanol (4 :1 by volume) or 0.15 with methylene chloride/ethanol (9 :1 by volume).

6. Urdamycin D, which has a melting point of 220 °C (with decomposition) ; a C content of about 64.1 %, an H content of about 6.5 % and an N content of about 1.5 % ; IR bands (in KBr) at 3420, 1735, 1710 (sh), 1690, 1668 and 1595 $cm^{-1}$ ; UV bands (in methanol with and without HCl) $\lambda_{max}(\varepsilon)$ 575 (11200) and 328 nm (11600) or (in methanol/NaOH) 635 (7200, sh), 600 (8500), 412 (4200), 324 (7500), 270 (17600, sh) and 222 nm (32400) ; a circular dichroism spectrum in methanol $\lambda_{extr.}(\theta^{24} \times 10^{-4}) = 440$ (+ 0.1), 395 (— 0.3), 350 (+ 1.0), 320 sh (— 0.4), 278 (— 1.5), 245 (+ 2.0) and 226 nm (+ 5.9) ; and an $R_f$ value on silica gel thin layer plates (20 × 20 cm, 0.25 mm layer thickness, development distance 15 cm) of 0.52 with chloroform/methanol (4 :1 by volume) or 0.17 with methylene chloride/ethanol (9 :1 by volume).

7. Urdamycin A, B, C, D, E or F as claimed in any one of claims 1 to 6, obtainable by aerobic fermentation of Streptomyces fradiae DSM 3093 and isolation from the culture medium.

8. Urdamycin A, B, C, D, E or F as claimed in claim 1 for which the isolation is carried out with the aid of

a) extraction of the culture medium with an organic solvent which has a low water-miscibility or is water-immiscible,

b) removal of strongly lipophilic constituents and

c) chromatography on silica gel using a mixture of a lower chlorohydrocarbon as the main

constituent, and a lower alkanol as the minor constituent, as the mobile phase, the urdamycins being isolated in the sequence B, E, A, D, C and F.

9. A process for the preparation of a compound with an antibiotic action by aerobic fermentation of Streptomyces, which comprises fermenting the strain S. fradiae DSM 3093, extracting the antibiotically active components from the culture medium and, if appropriate, fractionating them.

10. Streptomyces fradiae DSM 3093.

11. The use of an urdamycin as claimed in any one of claims 1 to 8 or of a compound obtained as claimed in claim 9 for the preparation of a medicine.

12. The use of urdamycin A as claimed in claim 3 for the preparation of aquayamycin.

13. The use of urdamycin B, C, D, E or F as claimed in any one of claims 4 to 8 for the preparation of an aglycone.

14. A process for the preparation of urdamycinone B, C, D, E or F, which comprises splitting off the O-glycosidically bonded sugars from urdamycin B, C, D, E or F as claimed in any one of claims 4 to 8 under acid conditions.

15. Urdamycinone B of the formula V

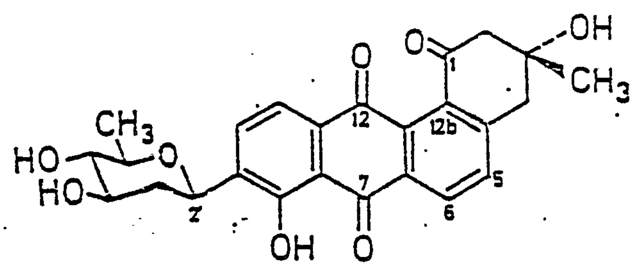

(V)

16. Urdamycinone E of the formula VI

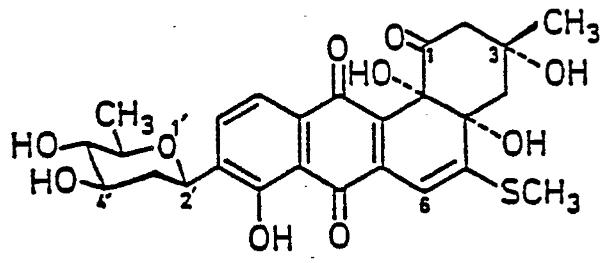

(VI)

17. Urdamycinone F of the formula VII

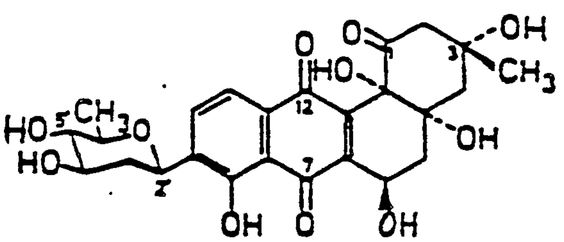

(VII)

18. Urdamycinone C, obtainable from urdamycin C as claimed in claim 7 by splitting off the O-glycosidically bonded sugar molecules under acid conditions.

19. Urdamycinone D, obtainable from urdamycin D as claimed in claim 8 by splitting off the O-glycosidically bonded sugar molecules under acid conditions.

20. Urdamycinone C, which has a melting point above 350 °C ; a C content of about 63.6 % and an H content of about 5.5 % ; IR bands (in KBr) at 3420, 1726, 1708 (sh), 1640 and 1604 $cm^{-1}$ ; UV bands (in methanol, with and without HCl) $\lambda_{max}(\varepsilon)$ 513 (13200), 311 (15700) and 227 nm (28000) or (in methanol/NaOH) 630 (11500, sh) 600 (12400), 412 (5000), 323 (9600) and 240 nm (30400) ; a circular dichroism spectrum in methanol $\lambda_{extr.}(\theta^{24} \times 10^{-4})$ = 520 (+ 0.6), 420 (0), 340 (+ 1.0), 314 (— 0.6), 295 (0), 275 (— 0.6), 246 (+ 0.9), 239 (+ 0.7) and 221 nm (+ 3.8) ; and an $R_f$ value on silica gel thin layer plates (20 × 20 cm, 0.25 mm layer thickness, development distance 15 cm) of 0.52 with chloroform/methanol (4 :1 by volume) or 0.15 with methylene chloride/ethanol (9 :1 by volume).

21. The use of urdamycinone B, C, D, E or F as claimed in any one of claims 15 to 20 for the preparation of a medicine.

22. Urdamycin A, B, C, D, E or F as claimed in any one of claims 3 to 8 and urdamycinone B, C, D, E or F as claimed in any one of claims 15 to 20 for use in medical treatment.

**Claims** (for the Contracting State AT)

1. A process for the preparation of a compound with an antibiotic action by aerobic fermentation of Streptomyces, which comprises
   a) fermenting the strain Streptomyces fradiae DSM 3093 and
   b) extracting the urdamycins from the culture medium.

2. A process for the preparation of a compound with an antibiotic action by aerobic fermentation of Streptomyces, which comprises
   a) fermenting the strain Streptomyces fradiae DSM 3093,
   b) extracting the urdamycins from the culture medium and
   c) fractionating the urdamycins.

3. The process as claimed in claim 2, wherein the extraction of the culture medium is carried out with an organic solvent which has a low water-miscibility or is water-immiscible, strongly lipophilic constituents are removed from the extract and the urdamycins are eluted in the sequence

Urdamycin B of the formula II

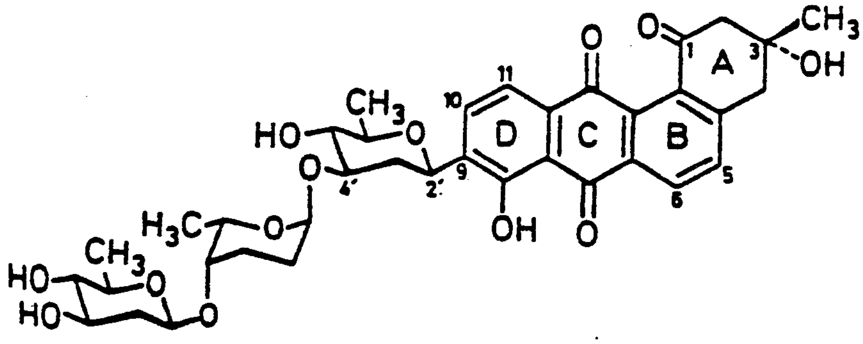

(II)

Urdamycin E of the formula III

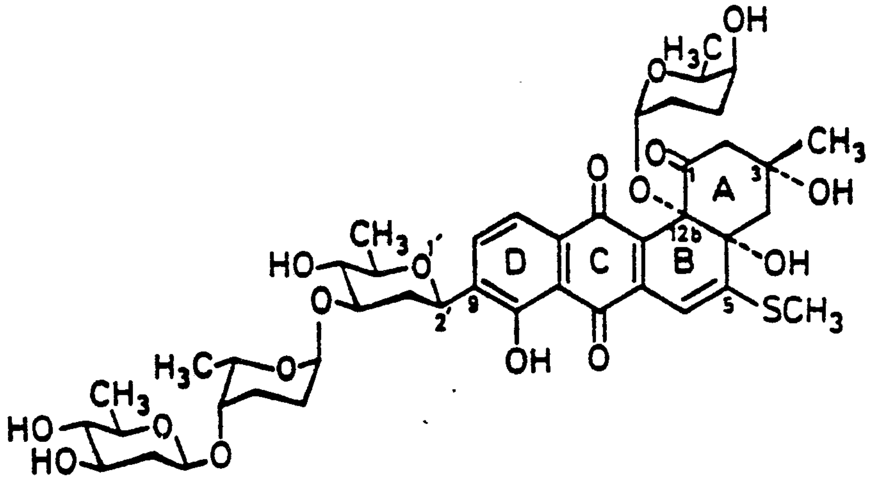

(III)

Urdamycin A of the formula I

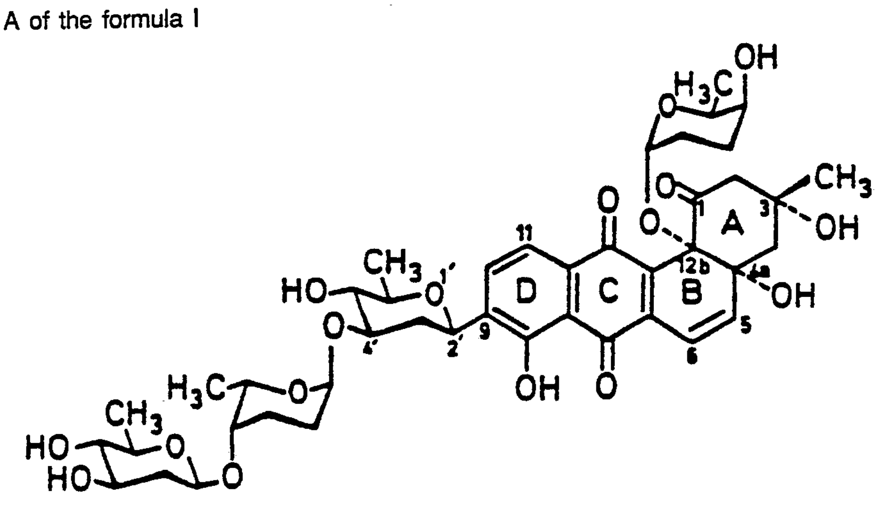

(I)

Urdamycin D, which has a melting point of 220 °C (with decomposition) ; a C content of about 64.1 %, an H content of about 6.5 % and an N content of about 1.5 % ; IR bands (in KBr) at 3420, 1735, 1710 (sh), 1690, 1668 and 1595 $cm^{-1}$ ; UV bands (in methanol with and without HCl) $\lambda_{max}(\varepsilon)$ 575 (11200) and 328 nm (11600) or (in methanol/NaOH) 635 (7200, sh), 600 (8500), 412 (4200), 324 (7500), 270 (17600, sh) and 222 nm (32400) ; a circular dichroism spectrum in methanol $\lambda_{extr.}(\theta^{24} \times 10^{-4})$ = 440 (+ 0.1), 395 (— 0.3), 350 (+ 1.0), 320 sh (— 0.4), 278 (— 1.5), 245 (+ 2.0) and 226 nm (+ 5.9) ; and an $R_f$ value on silica gel thin layer plates (20 × 20 cm, 0.25 mm layer thickness, development distance 15 cm) of 0.52 with chloroform/methanol (4 :1 by volume) or 0.17 with methylene chloride/ethanol (9 :1 by volume), Urdamycin C, which has a melting point of 200 °C (with decomposition) ; a C content of about 63.4 %, an H content of about 6.7 % ; IR bands (in KBr) at 3420, 1732, 1705 (sh), 1640 and 1605 $cm^{-1}$ ; UV bands (in methanol) $\lambda_{max}(\varepsilon)$ 510 (12600), 410 (6300, sh), 308 (14800), 237 (23600, sh) and 226 nm (25300) or (in methanol/HCl) $\lambda_{max}(\varepsilon)$ 510 (12600), 310 (15400) and 226 nm (26900) or (in methanol/NaOH) $\lambda_{max}(\varepsilon)$ 625 (10400, sh), 600 (12100), 414 (5500), 326 (12000) and 238 nm (30000) ; a circular dichroism spectrum in methanol $\lambda_{extr.}(\theta^{21} \times 10^{-4}$ = 500 (— 0.5), 336 (+ 1.7), 283 (— 1.2), 248 (+ 2.9), 236 (+ 2.2) and 223 nm (+ 4.8) ; and an $R_f$ value on silica gel thin layer plates (20 × 20 cm, 0.25 mm layer thickness, development distance 15 cm) of 0.51 with chloroform/methanol (4 :1 by volume) or 0.15 with methylene chloride/ethanol (9 :1 by volume) and

Urdamycin F of the formula IV

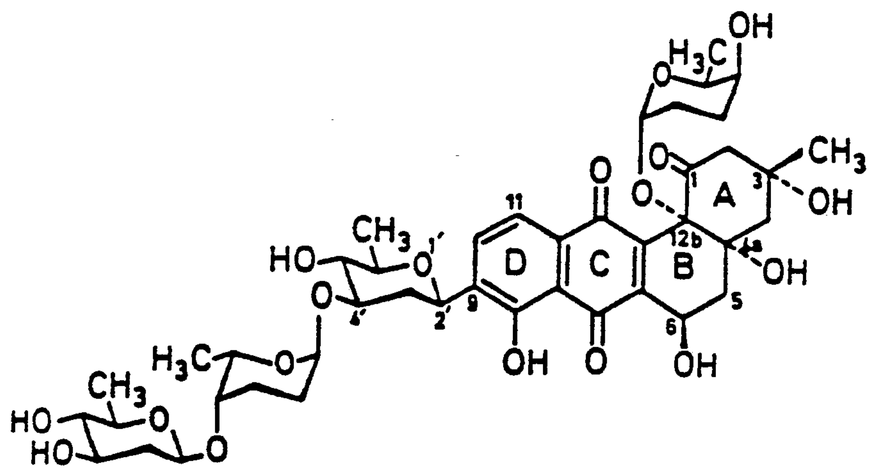

(IV)

by chromatography on silica gel with an eluting agent which is composed of a lower chlorohydrocarbon as the main constituent, and a lower alkanol as the minor constituent.

4. A process for the preparation of urdamycinone B, C, D, E or F, which comprises splitting off the O-glycosidically bonded sugars from urdamycin B, C, D, E or F as claimed in claim 3 under acid conditions to give Urdamycinone B of the formula V

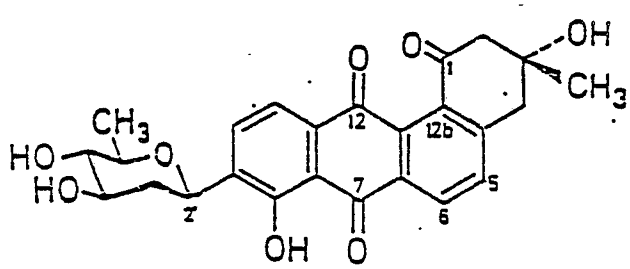

(V)

Urdamycinone E of the formula VI

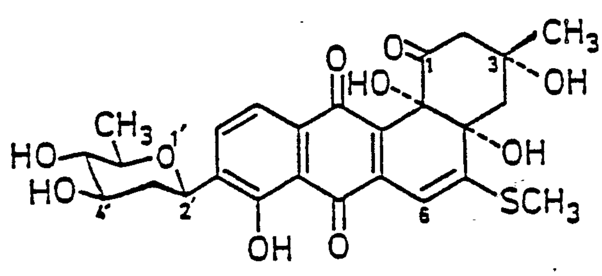

(VI)

Urdamycinone F of the formula VII

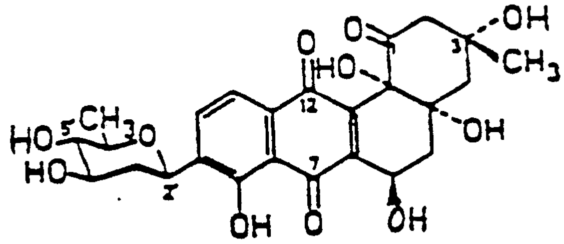

(VII)

Urdamycinone C, which has a melting point above 350 °C ; a C content of about 63.6 % and an H content of about 5.5 %, IR bands (in KBr) at 3420, 1726, 1708 (sh), 1640 and 1604 $cm^{-1}$ ; UV bands (in methanol, with and without HCl) $\lambda_{max}(\varepsilon)$ 513 (13200), 311 (15700) and 227 nm (28000) or (in methanol/NaOH) 630 (11500, sh) 600 (12400), 412 (5000), 323 (9600) and 240 nm (30400) ; a circular dichroism spectrum in methanol $\lambda_{extr.}(\theta^{24} \times 10^{-4})$ = 520 (+0.6), 420 (0), 340 (+1.0), 314 (—0.6), 295 (0), 275 (—0.6), 246 (+0.9), 239 (+0.7) and 221 nm (+3.8) ; and an $R_f$ value on silica gel thin layer plates (20 × 20 cm, 0.25 mm layer thickness, development distance 15 cm) of 0.52 with chloroform/methanol (4 :1 by volume) or 0.15 with methylene chloride/ethanol (9 :1 by volume) and urdamycinone D.

5. A process for the preparation of aquayamycin which comprises using urdamycin A as claimed in claim 3 as the starting product.

**Revendications** (pour les Etats contractants : BE, CH, DE, FR, GB, IT, LI, LU, NL, SE)

1. Urdamycine A de formule I

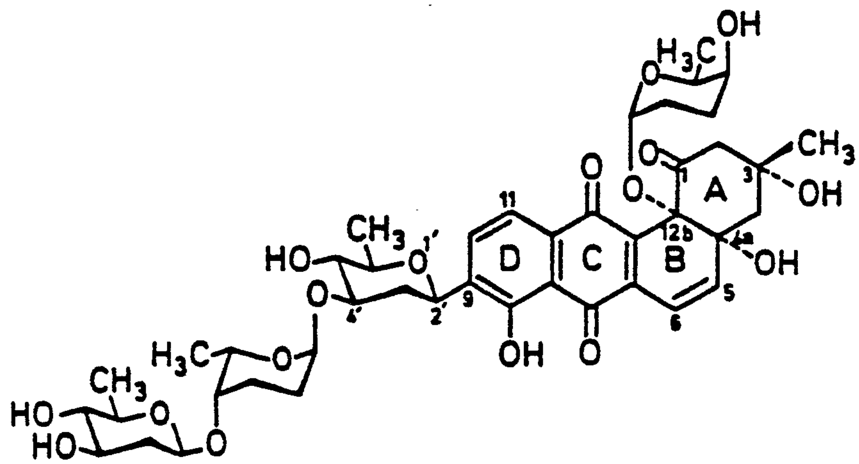

(I)

2. Urdamycine B de formule II

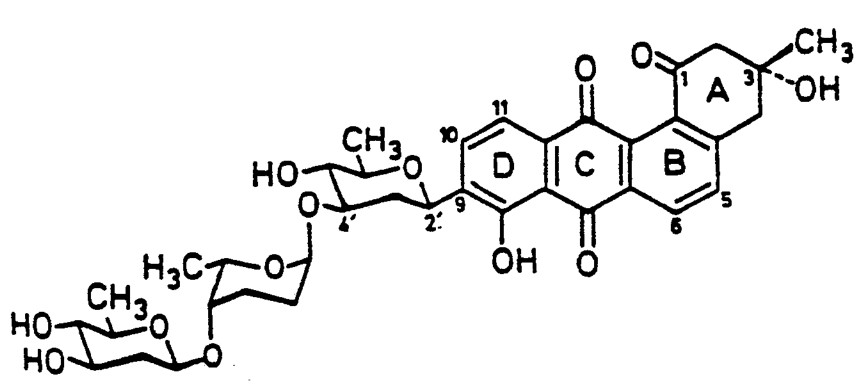

(II)

3. Urdamycine E de formule III

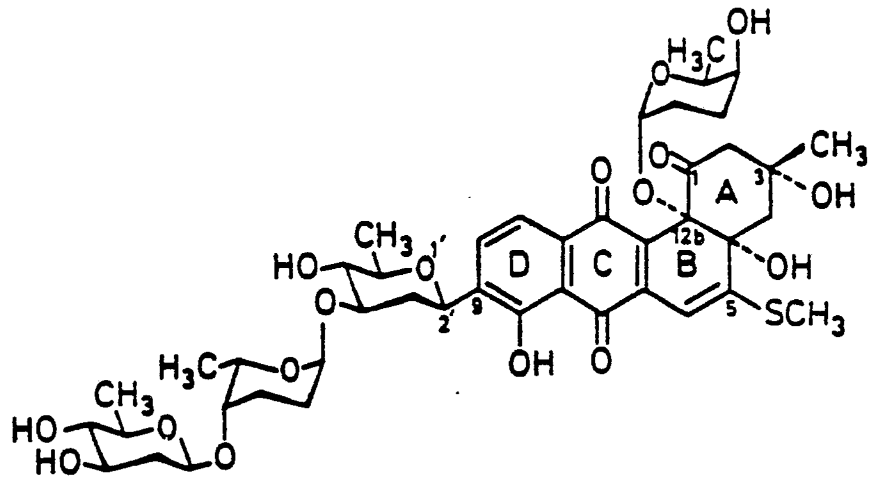

(III)

4. Urdamycine F de formule IV

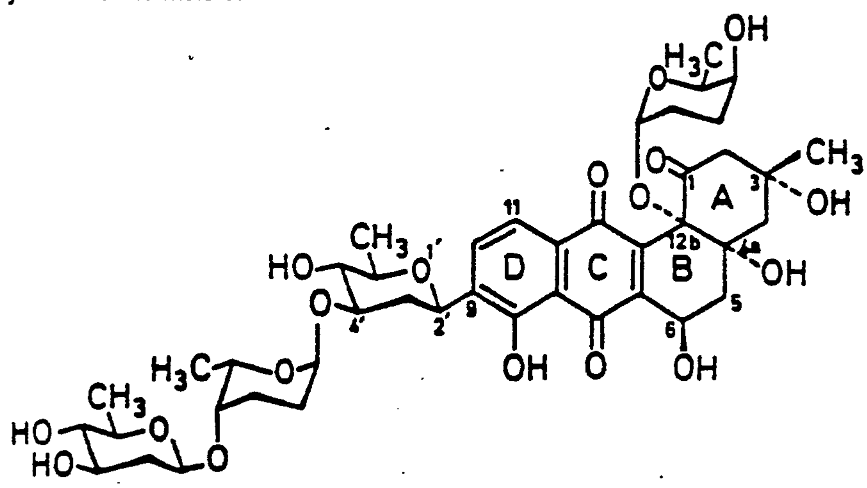

(IV)

5. Urdamycine C, caractérisée par un point de fusion de 200 °C (avec décomposition), une teneur en C d'environ 63,4 %, une teneur en H d'environ 6,7 %, des bandes IR (dans le KBr) à 3420, 1732, 1705 (ép.), 1640 et 1605 $cm^{-1}$, des bandes UV (dans le méthanol) $\lambda_{max}(\varepsilon)$ = 510 (12600), 410 (6300, ép.), 308 (14800), 237 (23600, ép.) et 226 nm (25300) ou (dans du méthanol/HCl) $\lambda_{max}(\varepsilon)$ = 510 (12600), 310 (15400) et 226 nm (26900) ou (dans du méthanol/NaOH) $\lambda_{max}(\varepsilon)$ = 625 (10400, ép.), 600 (12100), 414 (5500), 326 (12000) et 238 nm (30000), un spectre de dichroïsme circulaire dans le méthanol $\lambda_{extr.}(\theta^{21} \times 10^{4})$ = 500 (—0,5), 336 (+1,7), 283 (—1,2), 248 (+2,9), 236 (+2,2) et 223 nm (+4,8), ainsi qu'un $R_f$ sur plaques de chromatographie sur couche mince de gel de silice (20 × 20 cm, épaisseur de couche 0,25 mm, parcours 15 cm) = 0,51 avec un mélange de chloroforme/méthanol (4 :1 en volume) ou 0,15 avec un mélange de chlorure de méthylène/éthanol (9 :1 en volume).

6. Urdamycine D, caractérisée par un point de fusion de 220 °C (avec décomposition), une teneur en C d'environ 64,1 %, une teneur en H d'environ 6,5 % et une teneur en N d'environ 1,5 %, des bandes IR (dans le KBr) à 3420, 1735, 1710 (ép.), 1690, 1668 et 1595 $cm^{-1}$, des bandes UV (dans le méthanol, avec et sans HCl) $\lambda_{max}(\varepsilon)$ = 575 (11200) et 328 nm (11600) ou (dans du méthanol/NaOH) = 635 (7200, ép.), 600 (8500), 412 (4200), 324 (7500), 270 (17600, ép.) et 222 nm (32400), un spectre de dichroïsme circulaire dans le méthanol $\lambda_{extr.}(\theta^{24} \times 10^{-4})$ = 440 (+0,1), 395 (—0,3), 350 (+1,0), 320 ép. (—0,4), 278 (—1,5), 245 (+2,0) et 226 nm (+5,9), ainsi qu'un $R_f$ sur plaques de chromatographie sur couche mince de gel de silice (20 × 20 cm, épaisseur de couche 0,25 mm, parcours 15 cm) = 0,52 avec un mélange de chloroforme/méthanol (4 :1 en volume) ou 0,17 avec un mélange de chlorure de méthylène/éthanol (9 :1 en volume).

7. Urdamycines A à F selon les revendications 1 à 6, pouvant être obtenues par fermentation aérobie de Streptomyces fradiae DSM 3093 et isolement à partir du milieu de culture.

8. Urdamycines A à F selon la revendication 1, caractérisée en ce que l'isolement est effectué à l'aide

a) d'une extraction du milieu de culture avec un solvant organique peu miscible ou non miscible à l'eau,

b) élimination de composants fortement lipophiles et

c) chromatographie sur gel de silice avec utilisation, comme éluant, d'un mélange d'un hydrocarbure chloré inférieur en tant que composant principal et d'un alcool inférieur en tant que composant secondaire, les urdamycines étant isolées dans l'ordre B, E, A, D, C et F.

9. Procédé pour la préparation de composés à activité antibiotique, par fermentation aérobie de Streptomyces, caractérisé en ce que l'on cultive par fermentation la souche S. fradiae DSM 3093, on extrait à partir du milieu de culture le composant à activité antibiotique, et éventuellement on le sépare.

10. Streptomyces fradiae DSM 3093.

11. Utilisation des urdamycines selon les revendications 1 à 8 ou des composés obtenus selon la revendication 9, pour la préparation de médicaments.

12. Utilisation de l'urdamycine A selon la revendication 3 pour la préparation de l'aquayamycine.

13. Utilisation des urdamycines B à F selon les revendications 4 à 8, pour la préparation des aglycones.

14. Procédé pour la préparation des urdamycinones B à F, caractérisé en ce que l'on élimine par voie acide les sucres liés par une liaison O-glycosidique, hors des urdamycines A à F selon les revendications 4 à 8.

15. Urdamycinone B de formule V

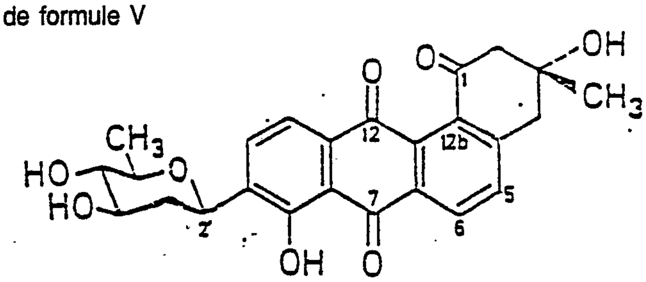

(V)

16. Urdamycinone E de formule VI

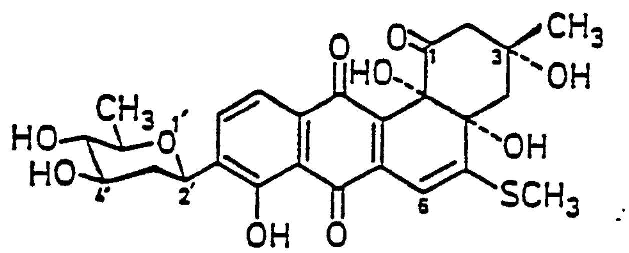

(VI)

17. Urdamycinone F de formule VII

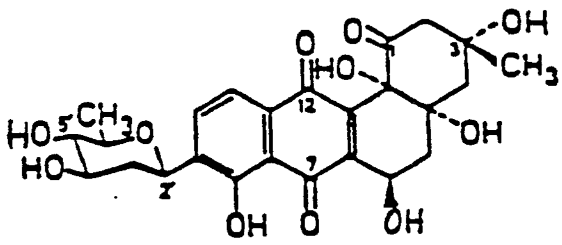

(VII)

18. Urdamycinone C préparable à partir de l'urdamycine C selon la revendication 7, par élimination par voie acide des molécules de sucres liées par une liaison O-glycosidique.

19. Urdamycinone D préparable à partir de l'urdamycine D selon la revendication 8, par élimination par voie acide des molécules de sucres liées par une liaison O-glycosidique.

20. Urdamycinone C, caractérisée par un point de fusion de plus de 350 °C ; une teneur en C d'environ 63,6 % et une teneur en H d'environ 5,5 % ; des bandes IR (dans le KBr) à 3420, 1726, 1708 (ép.), 1640 et 1604 $cm^{-1}$ ; des bandes UV (dans le méthanol, avec et sans HCl) $\lambda_{max}(\varepsilon)$ = 513 (13200), 311 (15700) et 227 nm (28000) ou (dans du méthanol/NaOH) = 630 (11500, ép.), 600 (12400), 412 (5000), 323 (9600) et 240 nm (30400) ; un spectre de dichroïsme circulaire dans le méthanol $\lambda_{extr.}(\theta^{24} \times 10^{-4})$ = 520 (+0,6), 420 (0), 340 (+1,0), 314 (—0,6), 295 (0), 275 (—0,6), 246 (+0,9), 239 (+0,7) et 221 nm (+3,8) ainsi qu'un $R_f$ sur plaques de chromatographie sur couche mince de gel de silice (20 × 20 cm, épaisseur de couche 0,25 mm, parcours 15 cm) = 0,52 avec un mélange de chloroforme/méthanol (4 :1 en volume) ou 0,15 avec un mélange de chlorure de méthylène/éthanol (9 :1 en volume).

21. Utilisation des urdamycinones B à F selon les revendications 15 à 20, pour la fabrication de médicaments.

22. Urdamycines A à F selon les revendications 3 à 8 et urdamycinones B à F selon les revendications 15 à 20, pour l'utilisation en thérapeutique.

**Revendications** (pour l'Etat contractant AT)

1. Procédé pour la préparation de composés à activité antibiotique, par fermentation aérobie de Streptomyces, caractérisé en ce que
a) on cultive par fermentation la souche Streptomyces fradiae DSM 3093 et
b) on extrait les urdamycines hors du milieu de culture.

2. Procédé pour la préparation de composés à activité antibiotique, par fermentation aérobie de Streptomyces, caractérisé en ce que
a) on cultive par fermentation la souche Streptomyces fradiae DSM 3093,
b) on extrait les urdamycines hors du milieu de culture et
c) on sépare les urdamycines.

3. Procédé selon la revendication 2, caractérisé en ce que l'on effectue l'extraction du milieu de culture avec un solvant organique qui est peu ou non miscible à l'eau, on élimine hors de l'extrait les composants fortement lipophiles et on élue par chromatographie sur gel de silice, avec un éluant qui est composé d'un hydrocarbure chloré inférieur en tant que composant principal et d'un alcool inférieur en tant que composant secondaire, les urdamycines dans l'ordre urdamycine B de formule II,

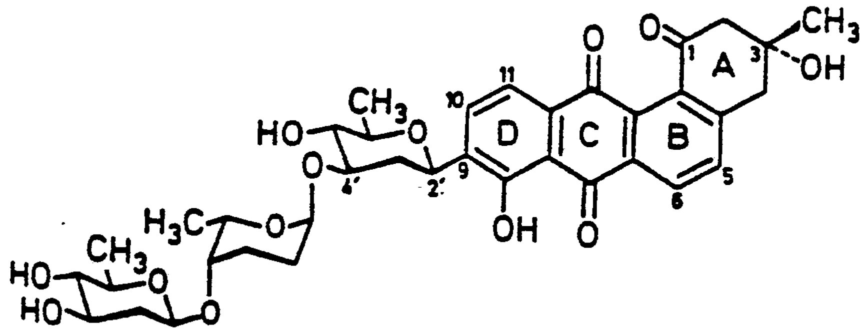

(II)

Urdamycine E de formule III,

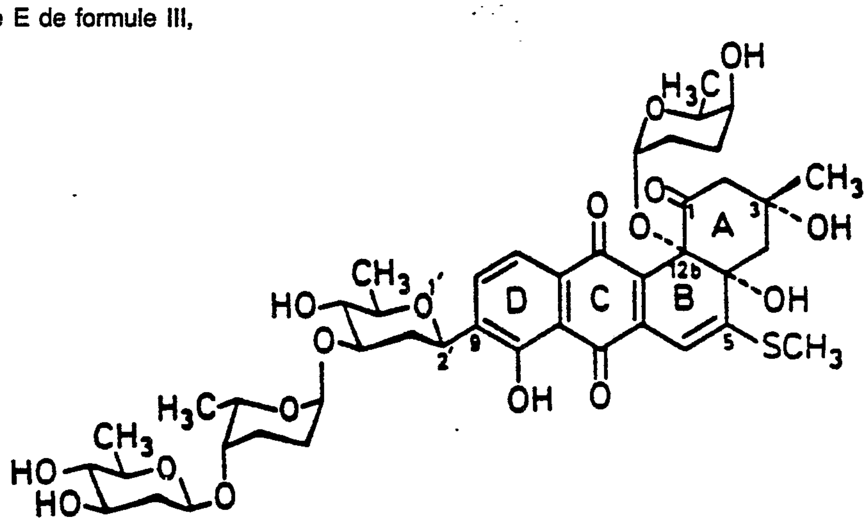

(III)

Urdamycine A de formule I,

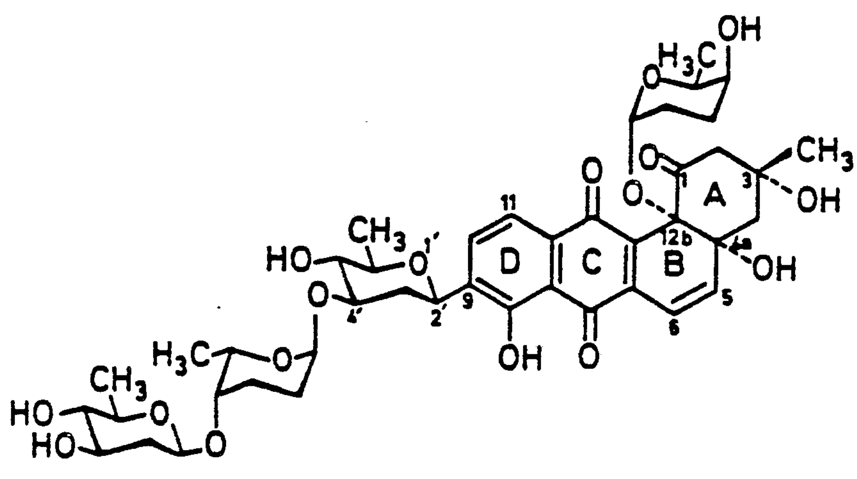

(I)

Urdamycine D, caractérisée par un point de fusion de 220 °C (avec décomposition), une teneur en C d'environ 64,1 %, une teneur en H d'environ 6,5 % et une teneur en N d'environ 1,5 %, des bandes IR (dans le KBr) à 3420, 1735, 1710 (ép.), 1690, 1668 et 1595 $cm^{-1}$, des bandes UV (dans le méthanol, avec et sans HCl) $\lambda_{max}(\varepsilon)$ = 575 (11200) et 328 nm (11600) ou (dans du méthanol/NaOH) = 635 (7200, ép.), 600 (8500), 412 (4200), 324 (7500), 270 (17600, ép.) et 222 nm (32400), un spectre de dichroïsme circulaire dans le méthanol $\lambda_{extr.}(\theta^{24} \times 10^{-4})$ = 440 (+0,1), 395 (—0,3), 350 (+1,0), 320 ép. (—0,4), 278 (—1,5), 245 (+2,0) et 226 nm (+5,9), ainsi qu'un $R_f$ sur plaque de chromatographie sur couche mince de gel de silice (20 × 20 cm, épaisseur de couche 0,25 mm, parcours 15 cm) = 0,52 avec un mélange de chloroforme/méthanol (4 :1 en volume) ou 0,17 avec un mélange de chlorure de méthylène/éthanol (9 :1 en volume), urdamycine C, caractérisée par un point de fusion de 200 °C (avec décomposition) ; une teneur en C d'environ 63,4 %, une teneur en H d'environ 6,7 %, des bandes IR (dans le KBr) à 3420, 1732, 1705 (ép.), 1640 et 1605 $cm^{-1}$ ; des bandes UV (dans le méthanol) $\lambda_{max}(\varepsilon)$ = 510 (12600), 410 (6300, ép.), 308 (14800), 237 (23600, ép.) et 226 nm (25300) ou (dans du méthanol/HCl) $\lambda_{max}(\varepsilon)$ = 510 (12600), 310 (15400) et 226 nm (26900) ou (dans du méthanol/NaOH) $\lambda_{max}(\varepsilon)$ = 625 (10400, ép.), 600 (12100), 414 (5500) 326 (12000) et 238 nm (30000), un spectre de dichroïsme circulaire dans le méthanol $\lambda_{extr.}(\theta^{21} \times 10^{-4})$ = 500 (—0,5), 336 (+1,7), 283 (—1,2), 248 (+2,9), 236 (+2,2) et 223 nm (+4,8), ainsi qu'un $R_f$ sur plaque de chromatographie sur couche mince de gel de silice (20 × 20 cm, épaisseur de couche 0,25 mm, parcours 15 cm) = 0,51 avec un mélange de chloroforme/méthanol (4 :1 en volume) ou 0,15 avec un mélange de chlorure de méthylène/éthanol (9 :1 en volume), et

Urdamycine F de formule IV

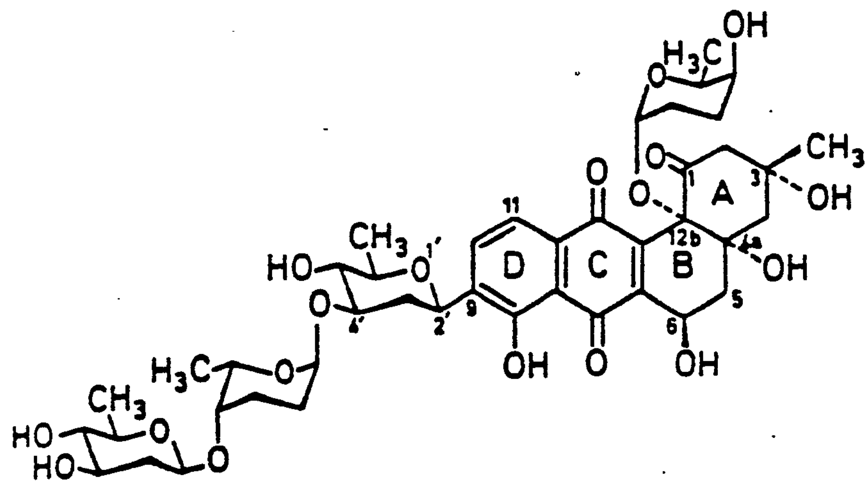

(IV)

4. Procédé pour la préparation des urdamycinones B à F, caractérisé en ce que l'on élimine par voie acide les sucres liés par une liaison O-glycosidique, hors des urdamycines A à F selon la revendication 3, et on obtient l'urdamycinone B de formule V

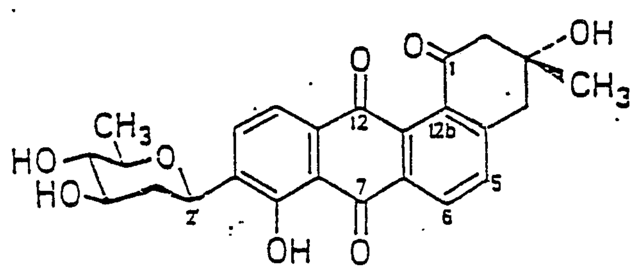

(V)

L'urdamycinone E de formule VI

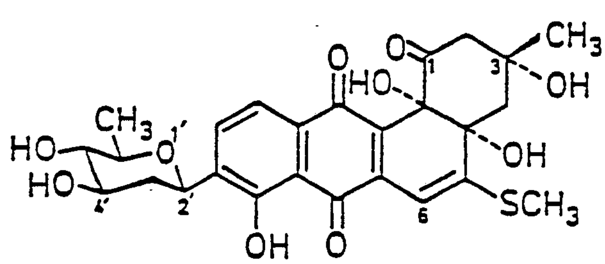

(VI)

L'urdamycinone F de formule VII

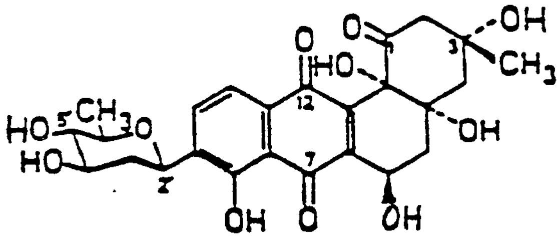

(VII)

L'urdamycinone C, caractérisée par un point de fusion de plus de 350 $_0$C ; une teneur en C d'environ 63,6 % et une teneur en H d'environ 5,5 % ; des bandes IR (dans le KBr) à 3420, 1726, 1708 (ép.), 1640 et 1604 $cm^{-1}$ ; des bandes UV (dans le méthanol, avec et sans HCl) $\lambda_{max}(\varepsilon)$ = 513 (13200), 311 (15700) et 227 nm (28000) ou (dans du méthanol/NaOH) = 630 (11500, ép.), 600 (12400), 412 (5000), 323 (9600) et 240 nm (30400) ; un spectre de dichroïsme circulaire dans le méthanol $\lambda_{extr.}(\theta^{24} \times 10^{-4})$ = 520 (+0,6), 420 (0), 340 (+1,0), 314 (—0,6), 295 (0), 275 (—0,6), 246 (+0,9), 239 (+0,7) et 221 nm (+3,8) ainsi qu'un $R_f$ sur plaques de chromatographie sur couche mince de gel de silice (20 × 20 cm, épaisseur de couche 0,25 mm, parcours 15 cm) = 0,52 avec un mélange de chloroforme/méthanol (4 :1 en volume) ou 0,15 avec un mélange de chlorure de méthylène/éthanol (9 :1 en volume), et l'urdamycinone D.

5. Procédé pour la préparation de l'aquayamycine, caractérisé en ce que l'on utilise comme produit de départ l'urdamycine A selon la revendication 3.